# EUROPEAN PATENT SPECIFICATION

(11) **EP 1 458 687 B1**
(45) Date of publication and mention of the grant of the patent: **11.01.2012**
(21) Application number: 02804468.3
(22) Date of filing: 27.11.2002
(51) Int. Cl.: C07D 239/32, C07D 239/38, C07D 239/47, C07D 239/50, C07D 239/52, C07D 239/56, C07D 279/06, A61K 31/506, A61K 31/295, A61P 29/00, C07D 401/06

(54) **PYRIMIDINE COMPOUNDS AS INTERLEUKIN-12 (IL-12) INHIBITORS**
PYRIMIDINVERBINDUNGEN ALS INTERLEUKIN-12 (IL-12) INHIBITOREN
COMPOSES DE PYRIMIDINE EN TANT QU'INHIBITEURS DE L'INTERLEUKINE-12 (IL-12)

(30) Priority: 30.11.2001 US 742; 10.07.2002 US 192347
(43) Date of publication of application: 22.09.2004
(73) Proprietor: Synta Pharmaceuticals Corporation, Lexington, MA 02421 (US)
(72) Inventor: ONO, Mitsunori, Lexington, MA 02421 (US); SUN, Lijun, Harvard, MA 01451 (US); PRZEWLOKA, Teresa, Tewksbury, MA 01876 (US); ZHANG, Shijie, Nashua, NII 03062 (US); KOSTIK, Elena, Arlington, MA 02474 (US); YING, Weiwen, Ayer, MA 01432 (US); WADA, Yumiko, Billerica, MA 01862 (US); KOYA, Keizo, Chestnut Hill, MA 02467 (US); WU, Yaming, Lexington, MA 02420 (US); ZHOU, Dan, Lexington, MA 02421 (US); TATSUTA, Noriaki, Lexington, MA 02420 (US)
(74) Representative: Schnappauf, Georg
(86) International application number: PCT/US2002/038161
(87) International publication number: WO 2003/047516

(56) References cited:
- WO-A-00/62778
- US-B1- 6 384 032
- S. NISHGAKI ET AL.: "synthesis of iminodipyrimidines." TETRAHEDRON LETTERS., vol. 7, 1969, pages 539-542, XP002315171 NLELSEVIER SCIENCE PUBLISHERS, AMSTERDAM.

## Description

### BACKGROUND

Interleukin-12 (IL-12) is a heterodimeric cytokine (p70) composed of two subunits (p35 and p40), and plays key roles in immune responses by bridging innate resistance and antigen-specific adaptive immunity. Trinchieri (1993) Immunol. Today 14: 335. For example, it promotes type 1 T helper cell (Th1) responses and, hence, cell-mediated immunity. Chan et al. (1991) J. Exp. Med. 173: 869; Seder et al. (1993) Proc. Natl. Acad. Sci. USA 90: 10188; Manetti et al. (1993) J. Exp. Med.177: 1199; and Hsieh et al. (1993) Science 260: 547. Overproduction of IL-12 causes excessive Th1 responses, and may result in inflammatory disorders, such as insulin-dependent diabetes mellitus, multiple sclerosis, rheumatoid arthritis, psoriasis, Crohn's disease, or sepsis. See, for example, Gately et al. (1998) Annu. Rev. Immunol. 16: 495; and Abbas et al. (1996) Nature 383: 787. Thus, inhibiting IL-12 overproduction is an approach to treat the just-mentioned diseases. Trembleau et al. (1995) Immmunol. Today 16: 383; and Adorini et al. (1997) Chem. Immunol. 68: 175. For example, overproduction of IL-12 and the resultant excessive Th1 type responses can be suppressed by modulating IL-12 production. A compound that down-regulates IL-12 production can be used for treating inflammatory diseases. Ma et al. (1998) Eur. Cytokine Netw.9: 54.

Further references are: WO00/62778, US 6384032 and NISHGAKI et al., "Tetrahedron Letters", vol. 7, 1969, pages 539-542.

### SUMMARY

In one aspect, this invention features pyrimidine compounds of formula (I): 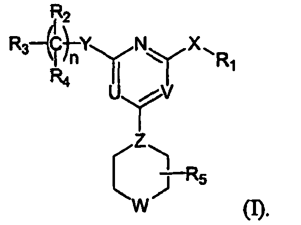 R₁ is 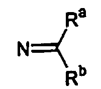 [referred to hereinafter as NC(R^{a}R^{b})]; each of R₂ and R₄, independently, is R^{c}, halogen, nitro, cyano, isothionitro, SR^{c}, or OR^{c}; or R₂ and R₄, taken together, is carbonyl; R₃ is R^{c}, alkenyl, alkynyl, OR^{c}, OC(O)R^{c}, SO₂R^{c}, S(O)R^{c}, S(O₂)NR^{c}R^{d}, SR^{c}, NR^{c}R^{d}, NR^{c}COR^{d}, NR^{c}C(O)OR^{d}, NR^{c}C(O)NR^{c}R^{d}, NR^{c}SO₂R^{d}, COR^{c}, C(O)OR^{c}, or C(O)NR^{c}R^{d}; R₅ is H or alkyl; n is 0, 1, 2, 3, 4, 5, or 6; X is O, S, S(O), S(O₂), or NR^{c}; Y is a covalent bond, CH₂, C(O), C=N-R^{c}, C=N-OR^{c}, C=N-SR^{c}, O, S, S(O), S(O₂), or NR^{c}; Z is N or CH; one of U and V is N, and the other is CR^{c}; and W is O, S, S(O), S(O₂), NR^{c}, or NC(O)R^{c}; in which each of R^{a} and R^{b}, independently, is H, alkyl, aryl, heteroaryl; and each of R^{c} and R^{d}, independently, is H, alkyl, aryl, heteroaryl, cyclyl, heterocyclyl, or alkylcarbonyl. Note that the left atom shown in any substituted group described above is closest to the pyrimidine ring. Also note that when n is 2 or greater, the just-described pyrimidine compound may have two or more different C(R²R⁴) moieties, or when there are more than one R^{c}-containing substituted groups in a pyrimidine compound, the R^{c} moieties can be the same or different. The same rules apply to other similar situations. Further note that R^{c} can be a monovalent or bivalent substitutent.

Referring to formula (I), in a subset of the pyrimidine compounds of this invention, U can be N, V can be CH, Z can be N, and W can be O. In addition, X can be O or NR^{c}; R^{c} can be H, methyl, ethyl, or acetyl; Y can be a covalent bond, O, S, or CH₂, and n can be 0, 1, 2, 3, or 4. In some embodiments, R₃ is aryl, heteroaryl (e.g., pyridinyl), OR^{c}, SR^{c}, C(O)OR^{c}, NR^{c}R^{d}, or C(O)NR^{c}R^{d}. In other embodiments, R₃ is 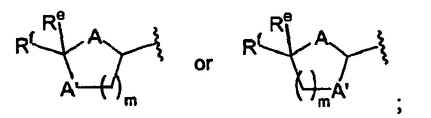 in which each of A and A', independently, is O, S, or NH; each of R^{c} and R^{f}, independently, is H, alkyl, aryl, or heteroaryl; and m is 1 or 2.

In this subset of pyrimidine compounds, R^{a} or R^{b}, preferably, is 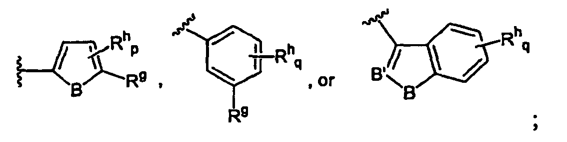 in which B is NRⁱ, O, or S; B' is N or CRⁱ; R^{g} is H, halogen, CN, alkyl, cyclyl, alkyloxy, alkylcarbonyl, alkyloxycarbonyl, aryloxycarbonyl, heteroaryloxycarbonyl, hydroxyalkyl, alkylamino, or alkylaminocarbonyl; R^{h} is H, halogen, NO₂, CN, alkyl, aryl, heteroaryl, OR^{c}, OC(O)R^{c}, SO₂R^{c}, S(O)Rⁱ, S(O₂)NR^{c}R^{d}, SR^{c}, NR^{c}R^{d}, NR^{c}COR^{d}, NR^{c}C(O)OR^{d}, NRC^{c}(O)NR^{d}, NR^{c}SO₂R^{d}, COR^{c}, C(O)OR^{c}, or C(O)NR^{c}R^{d}; Rⁱ is H, alkyl, or alkylcarbonyl; p is 0, 1, or 2; and q is 0, 1, 2, 3, or 4.

R^{a} or R^{b}, preferably, is 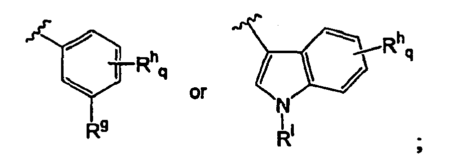 wherein R^{g} is H, methyl, ethyl, propyl, cyclopropyl, methoxy, ethoxy, halogen, or methoxycarbonyl; R^{h} is F, Cl, CN, methyl, methoxy, ethoxy, OC(O)CH₃, OC(O)C₂H₅, C(O)OH, C(O)OC₂H₅, C(O)NH₂, NHC(O)CH₃. or S(O₂)NH₂; Rⁱ is H, methyl, ethyl, or acetyl; and q is 0, 1, or 2.

Another subset of the pyrimidine compounds of formula (I) is featured by that each of R₂ and R₄ is H; R₃ is H, alkyl, aryl, heteroaryl, cyclyl, heterocyclyl, alkyloxycarbonyl, alkylaminocarbonyl, or alkylcarbonyl; R₅ is H or alkyl; n is 0, 1, 2, 3, 4, 5, or 6; X is NR^{c}; Y is covalent bond, CH₂, C(O), C=N-R^{c}, C=N-OR^{c}, C=N-SR^{c}, O, S, S(O), S(O₂), or NR^{c}; Z is N or CH; one ofU and V is N, and the other is CR^{c}; and W is O, S, S(O), S(O₂), NR^{c}, or NC(O)R^{c}; in which each of R^{a} and R^{b}, independently, is H, alkyl, aryl, heteroaryl; and R^{c} is H, alkyl, aryl, heteroaryl, cyclyl, heterocyclyl, or alkylcarbonyl.

In this subset of pyrimidine compounds, preferably, one of R^{a} and R^{b} is H or alkyl; and the other is aryl or heteroaryl optionally substituted with R^{g} and R^{h}_{q}; R^{g} being halogen, CN, alkyl, alkyloxy, alkylcarbonyl, alkyloxycarbonyl, aryloxycarbonyl, heteroaryloxycarbonyl, hydroxyalkyl, alkylamino, or alkylaminocarbonyl; R^{h} being halogen, CN, hydroxyl, alkyl, aryl, heteroaryl, alkoxyl, aryloxyl, or heteroaryloxyl; and q being 0, 1, 2, 3, or 4. Preferably, one of R^{a} and R^{b} is H or alkyl; and the other is 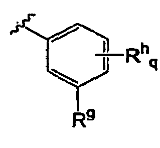 (e.g., 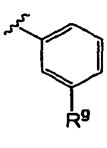 such as 3-methylphenyl); in which R^{g} is H, alkyl, alkoxyl, methoxycarbonyl, or halogen; R^{h} is halogen, CN, hydroxyl, alkyl, aryl, heteroaryl, alkoxyl, aryloxyl, or heteroaryloxyl; and q is 0, 1, 2, 3, or 4.

In some embodiments, X is NH; Y is O; n is 2, or R₃ is heteroaryl (e.g., pyridinyl or 1-oxy-pyridinyl) or heterocyclyl. (e.g., 1*H*-pyridin-2-one). In other embodiments, U is N; V is CH; and R₃ is heteroaryl or heterocyclyl. Preferably, X is NH; Y is O; n is 2; and one of R^{a} and R^{b} is H; and the other is 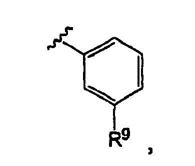 in which R^{g} can be CN, hydroxyalkyl, alkylamino, alkylaminocarbonyl alkyloxycarbonyl (e.g., C(O)OCH₃), or halogen (F, Cl, Br, or I) when R₃ is heteroaryl (e.g., pyridinyl), or R^{g} can be halogen (e.g., I), alkyl (e.g., methyl), or alkyloxycarbonyl (e.g., methoxycarbonyl) when R₃ is heterocyclyl (e.g., 1*H*-pyridin-2-one).

Alkyl, alkenyl, alkynyl, aryl, heteroaryl, cyclyl, and heterocyclyl mentioned above include both substituted and unsubstituted moieties. The term "substituted" refers to one or more substituents (which may be the same or different), each replacing a hydrogen atom. Examples of substituents include, but are not limited to, halogen (F, Cl, Br, or I), hydroxyl, amino, alkylamino, arylamino, dialkylamino, diarylamino, cyano, nitro, mercapto, carbonyl, carbamido, carbamyl, carboxyl, thioureido, thiocyanato, sulfoamido, alkyl, alkenyl, alkyloxy, aryl, heteroaryl, cyclyl, heterocyclyl, wherein alkyl, alkenyl, alkyloxy, aryl, heteroaryl cyclyl, and heterocyclyl are optionally substituted with alkyl, aryl, heteroaryl, halogen, hydroxyl, amino, mercapto, cyano, or nitro.

As used herein, the term "alkyl" refers to a straight-chained or branched alkyl group containing 1 to 6 carbon atoms. Examples of alkyl groups include methyl, ethyl, n-pmpyl, isopropyl, *tert*-butyl, and n-pentyl. Similarly, the term "alkenyl" or "alkynyl" refers to a straight-chained or branched alkenyl or alkynyl group containing up to 6 carbon atoms.

The term "aryl" refers to a hydrocarbon ring system (mono-cyclic or bi-cyclic) having at least one aromatic ring. Examples of aryl moieties include, but are not limited to, phenyl, naphthyl, and pyrenyl.

The term "heteroaryl" refers to a hydrocarbon ring system (mono-cyclic or bi-cyclic) having at least one aromatic ring which contains at least one heteroatom such as O, N, or S as part of the ring system and the reminder being carbon. Examples of heteroaryl moieties include, but are not limited to, furyl, pyrrolyl, thienyl, oxazolyl, imidazolyl, thiazolyl, pyridinyl, pyrimidinyl, quinazolinyl, and indolyl.

The terms "cyclyl" and "heterocyclyl" refer to a partially or fully saturated mono-cyclic or bi-cyclic ring system having from 4 to 14 ring atoms. A heterocyclyl ring contains one or more heteroatoms (e.g., O, N, or S) as part of the ring system and the remainder being carbon. Exemplary cyclyl and heterocyclyl rings are cycylohexane, piperidine, piperazine, morpholine, thiomorpholine, 1,4-oxazepane, and *1H*-pyridin-2-one.

The term "sulfanyl" refers to a thio group.

Set forth below are exemplary compounds of this invention:
N-{2-[3-(3,4-dimethoxy-phenyl)-propyl]-6-morpholin-4-yl-pyrimidin-4-yl}-N'-(1H-indol-3-ylmethylene)-hydrazine (Compound 1)
N-(2-n-butoxy-6-morpholin-4-yl-pyrimidin-4-yl)-N'-(1H-indol-3-ylmethylene)-hydrazine (Compound 2)
N-(2-(4-hydroxybutyl)-6-morpholin-4-yl-pyrimidin-4-yl)-N'-(1H-indol-3-ylmethylene)-hydrazine (Compound 3)
N-[2-(2-[1,3]dioxan-2-yl-ethyl)-6-morpholin-4-yl-pyrimidin-4-yl]-N'-(1H-indol-3-ylmethylene)-hydrazine (Compound 4)
N-(1H-indol-3-ylmethylene)-N'-[2-(3-methoxy-propyl)-6-morpholin-4-yl-pyrimidin-4-yl]-hydrazine (Compound 5)
3-{4-[N'-(1H-indol-3-ylmethylene)-hydrazino]-6-morpholin-4-yl-pyrimidin-2-ylsulfanyl}-propan-1-ol (Compound 6)
3-{2-[N'-(1H-indol-3-ylmethylene)-hydrazino]-6-morpholin-4-yl-pyrimidin-4-ylsulfanyl}-propan-1-ol (Compound 7)
N-[2-(2,2-dimethyl-[1,3]dioxolan-4-ylmethoxy)-6-morpholin-4-yl-pyrimidin-4-yl]-N'-(1H-indol-3-ylmethylene)-hydrazine (Compound 8)
N-{2-[2-(3,4-dirnethoxy-phenyl)-ethoxy]-6-morpholin-4-yl-pyrimidin-4-yl} - N'-(1H-indol-3-ylmethylene)-hydrazine (Compound 9)
N-(1H-indol-3-ylmethylene)-N'-[6-morpholin-4-yl-2-(2-pyridin-2-yl-ethoxy)-pyrimidin-4-yl]-hydrazine (Compound 10)
N-(1H-indol-3-ylmethylene)-N'-[6-morpholin-4-yl-2-(3-pyridin-2-yl-propyl)-pyrimidin-4-yl]-hydrazine (Compound 11)
N-(3-methyl-benzylidene)-N'-[6-morpholin-4-yl-2-(2-pyridin-2-yl-ethoxy)-pyrimidin-4-yl]-hydrazine (Compound 12)
N-(3-ethyl-benzylidene)-N'-[6-morpholin-4-yl-2-(2-pyridin-2-yl-ethoxy)-pyrimidin-4-yl]-hydrazine (Compound 13)
N-(3-methyl-benzylidene)-N'-[6-morpholin-4-yl-2-(3-pyridin-2-yl-propyl)-pyrimidin-4-yl]-hydrazine (Compound 14)
N-[6-morpholin-4-yl-2-(2-pyridin-2-yl-ethoxy)-pyrimidin-4-yl]-N'-(1-*m*-tolyl-ethylidene)-hydrazine (Compound 15)
N-[1-(1H-indol-3-yl)-ethylidene]-N'-[6-morpholin-4-yl-2-(2-pyridin-2-yl-ethoxy)-pyrimidin-4-yl]-hydrazine (Compound 16)
3-methyl-benzaldehyde O-[6-morpholin-4-yl-2-(2-pyridin-2-yl-ethoxy)-pyrimidin-4-yl]-oxime (Compound 17)
1H-indole-3-carbaldehyde O-[6-morpholin-4-yl-2-(2-pyridin-2-yl-ethoxy)-pyrimidin-4-yl]-oxime (Compound 18)
N-(1H-indol-3-ylmethylene)-N'-{6-morpholin-4-yl-2-[2-(pyridin-3-yloxy)-ethoxy]-pyrimidin-4-yl}-hydrazine (Compound 19)
N-(3-methyl-benzylidene)-N'-{6-morpholin-4-yl-2-[2-(pyridin-3-yloxy)-ethoxy]-pyrimidin-4-yl}-hydrazine (Compound 20)
butyl-{4-[N'-(1H-indol-3-ylmethylene)-hydrazino]-6-morpholin-4-yl-pyrimidin-2-yl}-amine (Compound 21)
N-(3-methyl-benzylidene)-N'-[6-morpholin-4-yl-2-(pyridin-3-yloxy)-pyrimidin-4-yl]-hydrazine (Compound 22)
N-(3-methylbenzlidene)-N'-(5-methyl-6-morpholin-4-yl-2-phenylpyrimidin-4-yl)hydrazine (Compound 23)
N-(3-methyl-benzylidene)-N'-(2-phenyl-6-thiomorpholin-4-yl-pyrimidin-4-yl)-hydrazine (Compound 24)
3-{4-[N'-(3-methyl-benzylidene)-hydrazino]-6-morpholin-4-yl-pyrimidin-2-yl}-propionic acid ethyl ester (Compound 25)
N-(3-methyl-benzylidene)-N'-{6-morpholin-4-yl-2-[2-(1-oxy-pyridin-2-yl)-ethoxy]-pyrimidin-4-yl}-hydrazine (Compound 26)
1-(2-{4-[N'-(3-methyl-benzylidene)-hydrazino]-6-morpholin-4-yl-pyrimidin-2-yloxy}-ethyl)-1H-pyridin-2-one (Compound 27)
N-(3-iodo-benzylidene)-N'-[6-morpholin-4-yl-2-(2-pyridin-2-yl-ethoxy)-pyrimidin-4-yl]-hydrazine (Compound 28)
N-(3-fluoro-benzylidene)-N'-[6-morpholin-4-yl-2-(2-pyridin-2-yl-ethoxy)-pyrimidin-4-yl]-hydrazine (Compound 29)
N-(3-chloro-benzylidene)-N'-[6-morpholin-4-yl-2-(2-pyridin-2-yl-ethoxy)-pyrimidin-4-yl]-hydrazine (Compound 30)
N-(3-bromo-benzylidene)-N'-[6-morpholin-4-yl-2-(2-pyridin-2-yl-ethoxy)-pyrimidin-4-yl]-hydrazine (Compound 31)
3-{[6-morpholin-4-yl-2-(2-pyridin-2-yl-ethoxy)-pyrimidin-4-yl]-hydrazonomethyl}-benzoic acid methyl ester (Compound 32)
1-(2-{4-[N'-(3-iodo-benzylidene)-hydrazino]-6-morpholin-4-yl-pyrimidin-2-yloxy}-ethyl)-1H-pyridin-2-one (Compound 33)
3-{[6-morpholin-4-yl-2-(2-pyridin-2-yl-ethoxy)-pyrimidin-4-yl]-hydrazonomethyl}-benzoic acid N-methyl amide (Compound 34)
(3-{[6-morpholin-4-yl-2-(2-pyridin-2-yl-ethoxy)-pyrimidin-4-yl]-hydrazonomethyl}-phenyl)-methanol (Compound 35)
N,N-diethyl-4-{4-[N"-(3-methyl-benzylidene)-hydrazino]-6-morpholin-4-yl-pyrimidin-2-yl}-butyramide (Compound 36)
4-{4-[N'-(3-methyl-benzylidene)-hydrazino]-6-morpholin-4-yl-pyrimidin-2-yl}-1-(4-methyl-piperazin-1-yl)-butan-1-one (Compound 37)
4-{4-[*N*'-(3-methyl-benzylidene)-hydrazino]-6-morpholin-4-yl-pyrimidin-2-yl}-*N*-pyridin-4-ylmethyl-butyramide (Compound 38)
4-{4-[*N*'-(3-methyl-benzylidene)-hydrazino]-6-morpholin-4-yl-pyrimidin-2-yl}-*N*-pyndin-4-yl-butyramide (Compound 39).

Their structures are depicted below:
Compound 1: 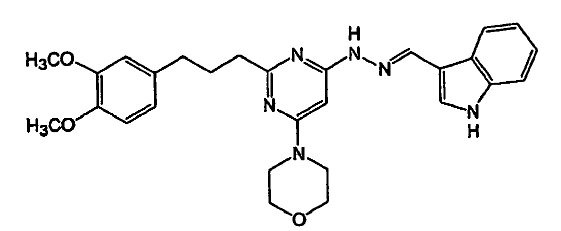
Compound 2: 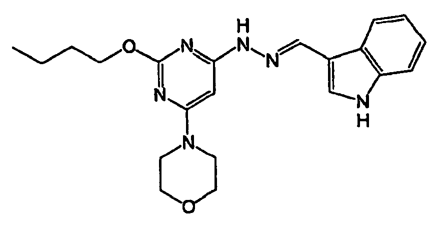
Compound 3: 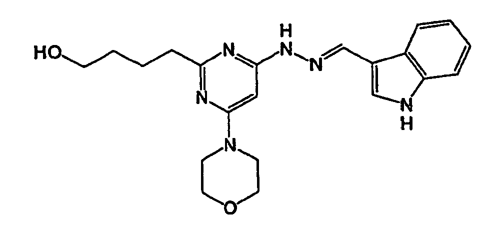
Compound 4: 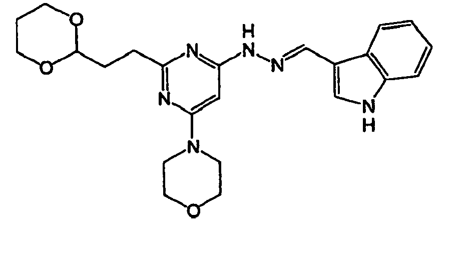
Compound 5: 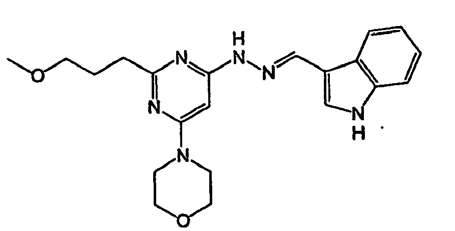
Compound 6: 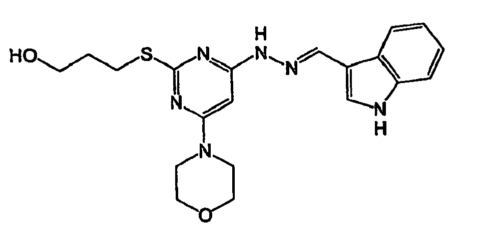
Compound 7: 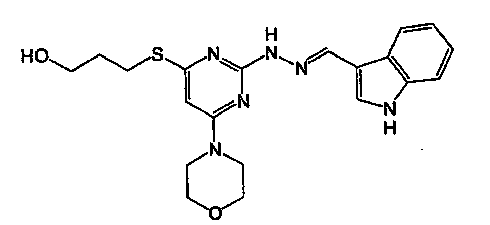
Compound 8: 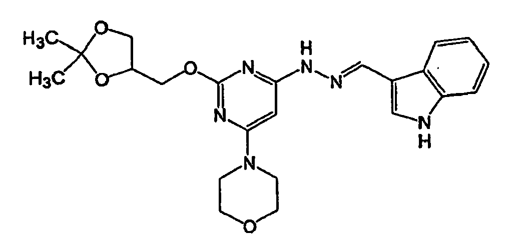
Compound 9: 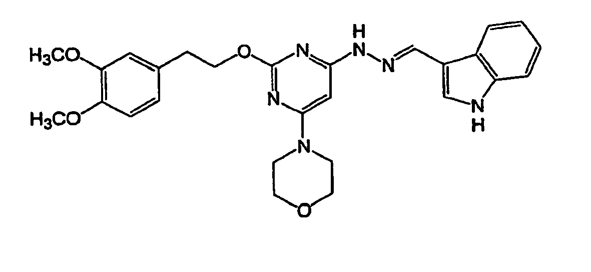
Compound 10: 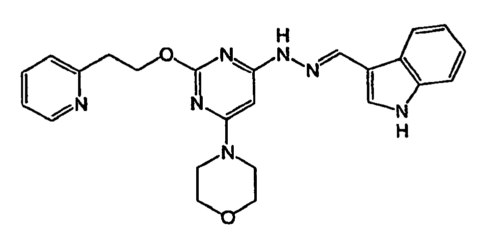
Compound 11: 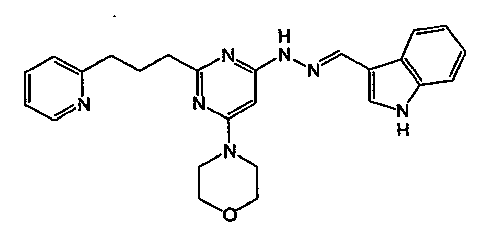
Compound 12: 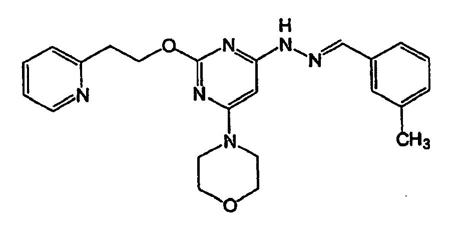
Compound 13: 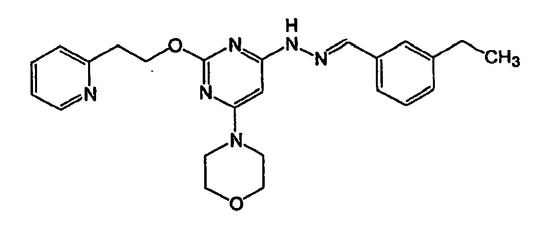
Compound 14: 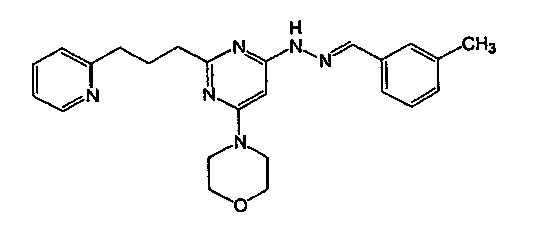
Compound 15: 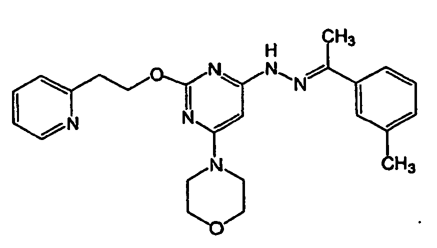
Compound 16: 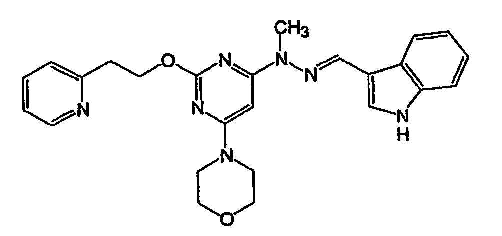
Compound 17: 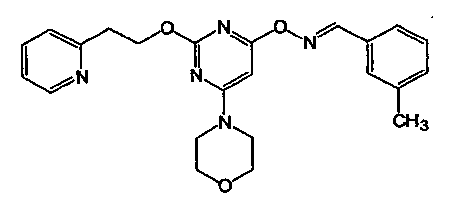
Compound 18: 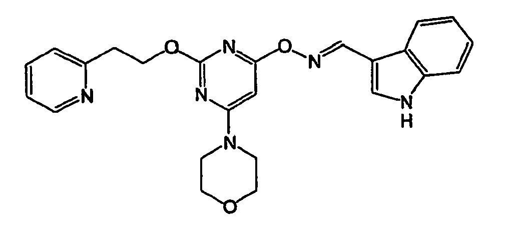
Compound 19: 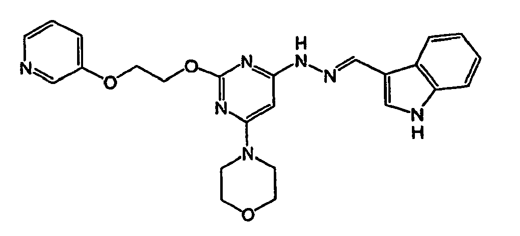
Compound 20: 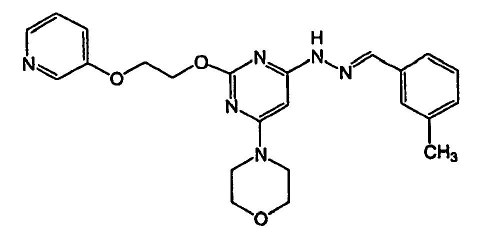
Compound 21: 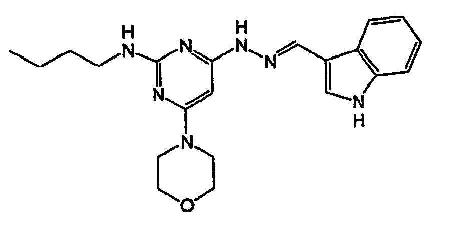
Compound 22: 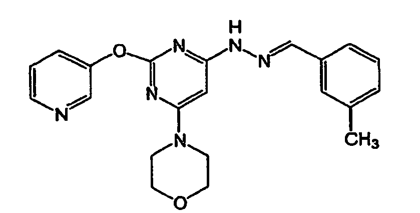
Compound 23: 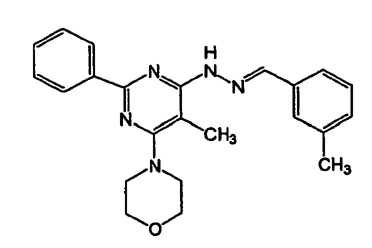
Compound 24: 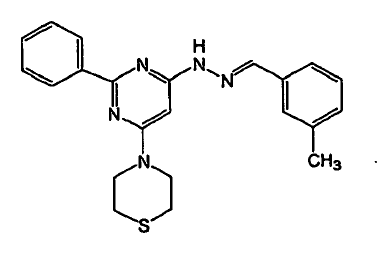
Compound 25: 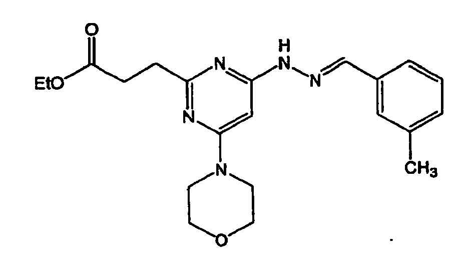
Compound 26: 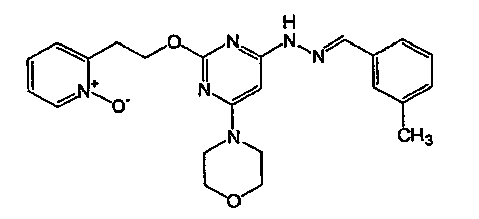
Compound 27: 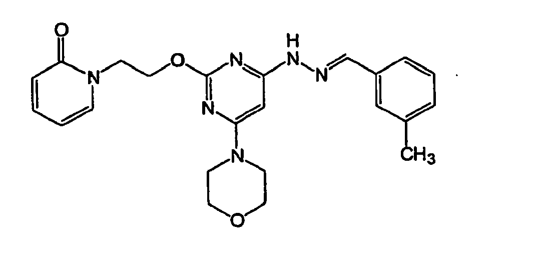
Compound 28: 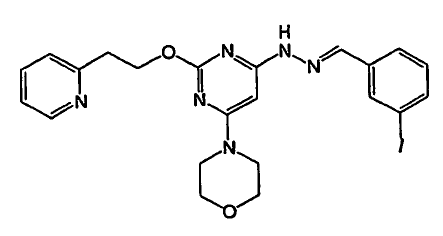
Compound 29: 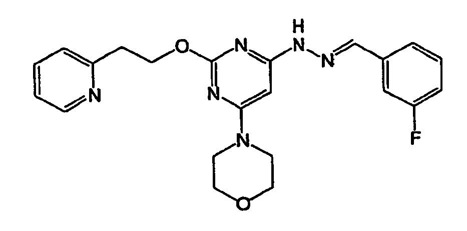
Compound 30: 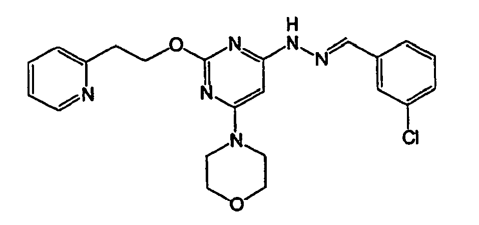
Compound 31: 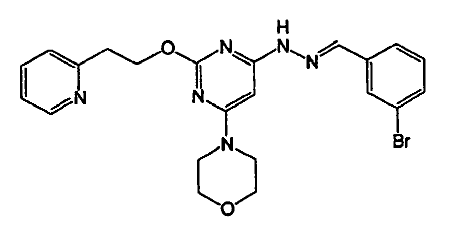
Compound 32: 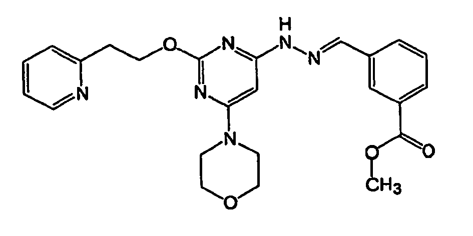
Compound 33: 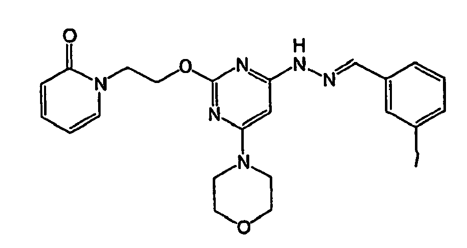
Compound 34: 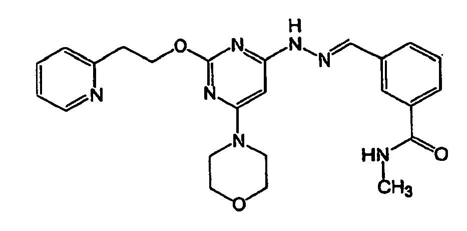
Compound 35: 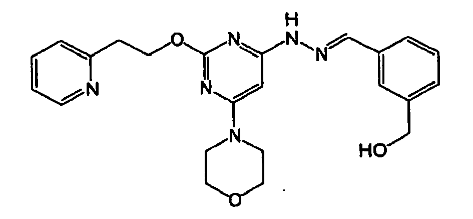
Compound 36: 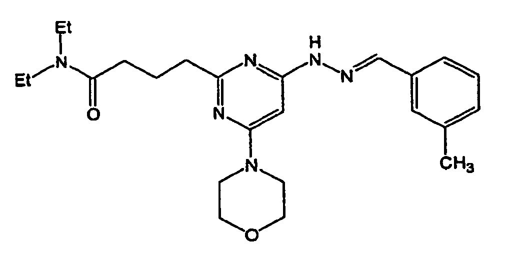
Compound 37: 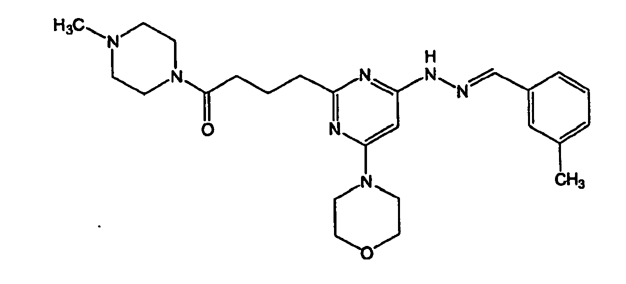
Compound 38: 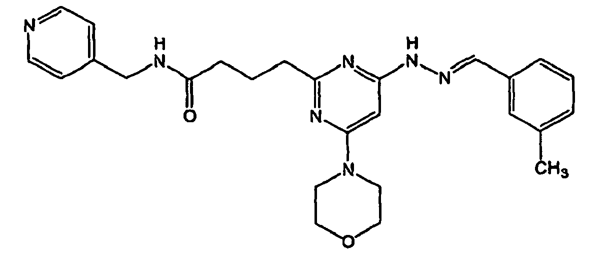
Compound 39:

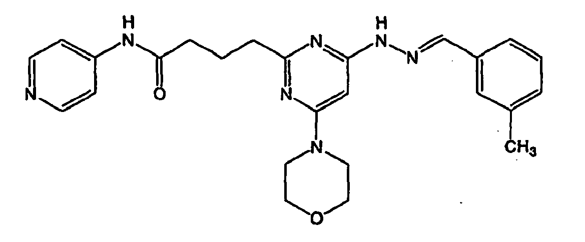

In another aspect, this invention features a pharmaceutical composition that contains a pharmaceutically acceptable carrier and an effective amount of at least one of the pyrimidine compounds of this invention.

In further another aspect, the present invention features a pyrimidine coumpound of this invention for use in a method for treating an IL-12 overproduction-related disorder (e.g., rheumatoid arthritis, sepsis, Crohn's disease, multiple sclerosis, psoriasis, or insulin-dependent diabetes mellitus). The method includes administering to a subject (e.g., a human or an animal) in need thereof an effective amount of one or more pyrimidine compounds of this invention. The method can also include the step of identifying that the subject is in need of treatment of diseases or disorders described above. The identification can be in the judgment of a subject or a health professional and can be subjective (e.g., opinion) or objective (e.g., measurable by a test or a diagnostic method).

The pyrimidine compounds of this invention include the compounds themselves, as well as their salts, if applicable. Such salts, for example, can be formed between a positively charged substituent (e.g., amino) on a compound and an anion. Suitable anions include, but are not limited to, chloride, bromide, iodide, sulfate, nitrate, phosphate, citrate, methanesulfonate, trifluoroacetate, and acetate. Likewise, a negatively charged substituent (e.g., carboxylate) on a compound can form a salt with a cation. Suitable cations include, but are not limited to, sodium ion, potassium ion, magnesium ion, calcium ion, and an ammonium cation such as teteramethylammonium ion.

In addition, some of the pyrimidine compounds of this invention have one or more double bonds, or one or more asymmetric centers. Such compounds can occur as racemates, racemic mixtures, single enantiomers, individual diastereomers, diastereomeric mixtures, and cis- or trans- or *E-* or *Z-* double isomeric forms.

Further, the aforementioned pyrimidine compounds also include their *N-*oxides. The term "*N-*oxides" refers to one or more nitrogen atoms, when present in a pyrimidine compound, are in *N-*oxide form, i.e., N→ O.

Combinations of substituents and variables envisioned by this invention are only those that result in the formation of stable pyrimidine compounds. The term "stable", as used herein, refers to compounds which possess stability sufficient to allow manufacture and which maintains the integrity of the compound for a sufficient period of time to be useful for the purposes detailed herein (e.g., treating IL-12 overproduction-related disorders such as rheumatoid arthritis, sepsis, Crohn's disease, multiple sclerosis, psoriasis, or insulin-dependent diabetes mellitus).

Also within the scope of this invention are a composition containing one or more of the pyrimidine compounds described above for use in treating an IL-12 overproduction-related disorder, and the use of such a composition for the manufacture of a medicament for the just-described use.

Other features, objects, and advantages of the invention will be apparent from the description and from the claims.

### DETAILED DESCRIPTION

The compounds described above can be prepared by methods well known in the art, as well as by the synthetic routes disclosed herein. For example, a pyrimidine compound can be prepared by using 2,4,6-trichloro-pyrimidine as a starting material. The three chloro groups can be displaced by various substitutes. More specifically, first chloro group (e.g., at position 6) can react with, e.g., morpholine, to form a morpholinyl pyrimidine. 2-Aryl and 2-alkylpyrimidine dichloro compounds can also be prepared by reacting an amidine with a malonic ester followed by treatment with phosphorous oxychloride. The second chloro group can be replaced by reacting with a nucleophile, such as an alcohol in the presence of base, e.g., sodium hydride. In other examples, a compound of formula (I), wherein Y is CH₂ (e.g., Compound 1), can be prepared by reacting the pyrimidine chloride with a Grignard reagent, an organotin reagent, an organocopper reagent, an organoboric acid, or an organozinc reagent in the presence of an organopalladium compound as a catalyst Isomeric forms may be produced. The desired isomeric product can be separated from others by, e.g., high performance liquid chromatography. The third chloro group undergoes a displacement reaction with, e.g., hydrazine, and the primary amine of the coupled hydrazine moiety further reacts with an aldehyde, e.g., indole-3-carboxaldehyde to form a hydrazone linkage. Thus, a pyrimidine compound of this invention is obtained. If preferred, other types of linkages can be prepared by similar reactions. Sensitive moieties on a pyrimidinyl intermediate and a nucleophile can be protected prior to coupling.

The chemicals used in the above-described synthetic routes may include, for example, solvents, reagents, catalysts, and protecting group and deprotecting group reagents. The methods described above may also additionally include steps, either before or after the steps described specifically herein, to add or remove suitable protecting groups in order to ultimately allow synthesis of the pyrimidine compounds. In addition, various synthetic steps may be performed in an alternate sequence or order to give the desired compounds. Synthetic chemistry transformations and protecting group methodologies (protection and deprotection) useful in synthesizing applicable pyrimidine compounds are known in the art and include, for example, those described in R. Larock, Comprehensive Organic Transformations, VCH Publishers (1989); T.W. Greene and P.G.M. Wuts, Protective Groups in Organic Synthesis, 3rd Ed., John Wiley and Sons (1999); L. Fieser and M. Fieser, Fieser and Fieser's Reagents for Organic Synthesis, John Wiley and Sons (1994); and L. Paquette, ed., Encyclopedia of Reagents for Organic Synthesis, John Wiley and Sons (1995) and subsequent editions thereof.

A pyrimidine compound thus obtained can be further purified by flash column chromatography, high performance liquid chromatography, or crystallization.

Also within the scope of this invention is a pharmaceutical composition that contains an effective amount of one or more of the pyrimidine compounds of this invention and a pharmaceutically acceptable carrier. Further, the present invention covers a pyrimidine compound of this invention for use in a method of administering an effective amount of such a compound to a subject in need of treatment of IL-12 overproduction related diseases (e.g., rheumatoid arthritis, sepsis, Crohn's disease, multiple sclerosis, psoriasis, or insulin-dependent diabetes mellitus). "An effective amount" refers to the amount of the compound which is required to confer a therapeutic effect on the treated subject. The interrelationship of dosages for animals and humans (based on milligrams per meter squared of body surface) is described in Freireich et al., (1966) Cancer Chemother. Rep.50: 219. Body surface area may be approximately determined from height and weight of the patient. See, e.g., Scientific Tables, Geigy Pharmaceuticals, Ardley, N.Y., 1970, 537. An effective amount of the pyrimidine compound of this invention can range from about 0.001 mg/Kg to about 1000 mg/Kg. Effective doses will also vary, as recognized by those skilled in the art, depending on the diseases treated, route of administration, excipient usage, and the possibility of co-usage with other therapeutic treatments such as use of other agents.

To practice the method described above, a pyrimidine compound, as a component of a pharmaceutical composition, can be administered orally, parenterally, by inhalation spray, topically, rectally, nasally, buccally, vaginally or via an implanted reservoir. The term "parenteral" as used herein includes subcutaneous, intracutaneous, intravenous, intramuscular, intraarticular, intraarterial, intrasynovial, intrasternal, intrathecal, intralesional and intracranial injection or infusion techniques.

A sterile injectable composition, for example, a sterile injectable aqueous or oleaginous suspension, can be formulated according to techniques know in the art using suitable dispersing or wetting agents (such as, for example, Tween 80) and suspending agents. The sterile injectable preparation can also be a sterile injectable solution or suspension in a non-toxic parenterally acceptable diluent or solvent, for example, as a solution in 1,3-butanediol. Among the acceptable vehicles and solvents that can be employed are mannitol, water, Ringer's solution and isotonic sodium chloride solution. In addition, sterile, fixed oils are conventionally employed as a solvent or suspending medium (e.g., synthetic mono- or diglycerides). Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions can also contain a long-chain alcohol diluent or dispersant, or carboxymethyl cellulose or similar dispersing agents. Other commonly used surfactants such as Tweens or Spans or other similar emulsifying agents or bioavailability enhancers which are commonly used in the manufacture of pharmaceutically acceptable solid, liquid, or other dosage forms can also be used for the purposes of formulation.

A composition for oral administration can be any orally acceptable dosage form including, but not limited to, capsules, tablets, emulsions and aqueous suspensions, dispersions and solutions. In the case of tablets for oral use, carriers which are commonly used include lactose and corn starch. Lubricating agents, such as magnesium stearate, are also typically added. For oral administration in a capsule form, useful diluents include lactose and dried corn starch. When aqueous suspensions or emulsions are administered orally, the active ingredient can be suspended or dissolved in an oily phase combined with emulsifying or suspending agents. If desired, certain sweetening, flavoring, or coloring agents can be added. A nasal aerosol or inhalation composition can be prepared according to techniques well-known in the art of pharmaceutical formulation and can be prepared as solutions in saline, employing benzyl alcohol or other suitable preservatives, absorption promoters to enhance bioavailability, fluorocarbons, and/or other solubilizing or dispersing agents known in the art. A pyrimidine compound of this invention can also be administered in the form of suppositories for rectal administration.

The carrier in the pharmaceutical composition must be "acceptable" in the sense of being compatible with the active ingredient of the formulation (and preferably, capable of stabilizing it) and not deleterious to the subject to be treated. For example, solubilizing agents such as cyclodextrins, which form specific, more soluble complexes with the compounds of this invention, or one or more solubilizing agents, can be utilized as pharmaceutical excipients for delivery of the pyrimidine compounds. Examples of other carriers include colloidal silicon dioxide, magnesium stearate, cellulose, sodium lauryl sulfate, and D&C Yellow # 10.

The biological activities of a pyrimidine compound can be evaluated by a number of cell-based assays. One of such assays can be conducted using cells from human peripheral blood mononuclear cells (PBMC) or human monocytic cell line (THP-1). The cells are stimulated with a combination of human interferon-γ (IFNγ) and lipopolysaccharide or a combination of IFNγ and *Staphylococcus aureus* Cowan I in the presence of a test compound. The level of inhibition of IL-12 production can be measured by determining the amount of p70 by using a sandwich ELISA assay with anti-human IL-12 antibodies. IC₅₀ of the test compound can then be determined. Specifically, PBMC or THP-1 cells are incubated with the test compound. Cell viability was assessed using the bioreduction of MTS [3-(4,5-dimethylthiazol-2-yl)-5-(3-carboxymethoxyphenyl)-2-(4-sulfophenyl)-2H-tetrazolium] (Promega, Madison, WI).

A pyrimidine compound can also be evaluated by animal studies. For example, one of such studies involves the ability of a test compound to treat adjuvant arthritis (i.e., a IL-12 overproduction-related disorder) in rats.

Without further elaboration, it is believed that the above description has adequately enabled the present invention. The following specific embodiments are, therefore, to be construed as merely illustrative, and not limitative of the remainder of the disclosure in any way whatsoever.

### Example 1. Preparation of Compound 1: N-{2-[3-(3,4-dimethoxy-phenyl)-propyl]-6-morpholin-4-yl-pyrimidin-4-yl}-N'-(1H-indol-3-ylmethylene)-hydrazine

To a solution of 3-(3,4-dimethoxyphenyl)-propyl iodide (1.224 g, 4.0 mmol) in 20 mL dry THF, highly active zinc (suspension in THF, Rieke metal from Aldrich, 5.2 mL 0.05g/mL, 4.0 mmol) was added to obtain a mixture. The mixture was stirred at room temperature overnight. 2,4-Dichloro-6-morpholinopyrimidine (0.932 g, 4.0 mmol) and *trans*-benzyl-(chloro)-bis-(triphenylphosphine)palladium(II) (0.03 g, 0.04 mmol) were added to the mixture, and stirred at 60°C for 2 days. After routine workup, 4-chloro-2-[3-(3,4-dimethoxyphenyl)propyl]-6-morpholinopyrimidine (0.34 g, 0.90 mmol, 22.4%) was separated from 2-chloro-4-[3-(3,4-dimethoxyphenyl)propyl]-6-morpholinopyrimidine (0.45 g, 1.19 mmol, 30%) by flash chromatography purification.

¹H NMR (300 MHz, CDCl₃), δ (ppm): 6.70-6.80 (m, 3H); 6.32 (s, 1H); 3.87 (s, 3H); 3.85 (s, 3H); 3.73-3.78 (m, 4H); 3.60-3.64 (m, 4H); 2.76 (d, *J* = 7.8 Hz, 2H); 2.63 (d, *J* = 7.5 Hz, 2H); and 2.01-2.12 (m, 2H).

MS (ESI): m/z 380.2 (M+H).

Further, 4-chloro-2-[3-(3,4-dimethoxyphenyl)propyl]-6-morpholinopyrimidine (0.34 g, 0.90 mmol) was reacted with hydrazine (0.29 g, 9 mmol) to obtain 2-[3-(3,4-dimethoxyphenyl)propyl]-4-hydrazino-6-morpholinopyrimidine as a white solid (0.30 g, 0.80 mmol, 89 %).

¹H NMR (300 MHz, CDCl₃), δ (ppm): 6.73-6.80 (m, 3H); 5.88 (s, 1H); 5.74 (s, 1H); 3.87 (s, 3H); 3.85 (s, 3H); 3.76-3.79 (m, 4H); 3.69 (d, *J* = 0.6 Hz, 2H); 3.56-3.60 (m, 4H); 2.64 (d, *J* = 7.5 Hz, 4H); and 2.00-2.15 (m, 2H).

MS (ESI): m/z 374.2 (M-H).

A 5 mL methanol solution containing 2-[3-(3,4-dimethoxyphenyl)-propyl]-4-hydrazino-6-morpholinopyrimidine (0.177 g, 0.50 mmol), indole-3-carboxaldehyde (0.073 g, 0.50 mmol), and AcOH (20 mg, cat.) was stirred at 70°C for 4 hours. Solvent was removed and the crude residue was purified using flash chromatography to give Compound 1 as a light brown solid (0.21 g, 0.42 mmol, 84 %).

¹H NMR (300 MHz, CDCl₃), δ (ppm): 8.57 (br s, 1H); 8.45 (br s, 1H); 8.29-8.32 (m, 1H); 8.00 (s, 1H); 7.39-7.43 (m, 2H); 7.23-7.34 (m, 2H); 6.74-6.80 (m, 3H); 6.30 (s, 1H); 3.86 (s, 3H); 3.85 (s, 3H); 3.78-3.84 (m, 4H); 3.67-3.70 (m, 4H); 2.63-2.71 (m, 4H), and 2.03-2.13 (m, 2H).

MS (ESI): m/z 501.2 (M+H).

### Example 2. Preparation of Compound 2: N-(2-n-butoxy-6-morpholin-4-yl-pyrimidin-4-yl)-N'-(1H-indol-3-ylmethylene)-hydrazine

To a solution of 2,4,6-trichloro-pyrimidine (25 g, 136 mmol) in CH₂Cl₂ (500 mL) at -78°C, morpholine (11.89 mL, 136 mmol) was slowly added, followed by DIPEA (25 mL, 143 mmol). The obtained reaction mixture was stirred at -78°C for 5 h, and then warmed up to room temperature. The reaction mixture was washed with water. The obtained organic phase was dried over Na₂SO₄. The solvent was removed under reduced pressure. The crued residue, 2,4-dichloro-6-(morpholin-4-yl)pyrimidine, was recrystallized from EtOAc to give white crystals (24.7 g, 77%) 15g.

¹H NMR (300 MHz, CDCl₃), δ (ppm): 6.40 (s, 1H); and 4.0 - 3.5 (m, 8H).

MS (ESI): m/z 234.0 (M+H).

To a solution of n-butanol (0.633 g, 8.54 mmol) in anhydrous DMF (50 mL) at 0°C under N₂, NaH (0.307 g, 12.8 mmol) was added quickly. The obtained suspension was stirred for 0.5 h at 0°C. 2,4-Dichloro-6-(morpholin-4-yl)pyrimidine (2 g, 8.54 mmol) was added to the suspension. After the suspension was warmed to room temperature and stirred for 12 h, the reaction mixture was quenched with ice/brine and extracted with 200 mL EtOAc. The extract was washed with brine, and dried over Na₂SO₄. The solvent was removed under reduced pressure. The crude residue was purified using flash chromatography (silica; EtOAc/hexane : 1/6) to yield 1.4 g of 2-n-butoxy-4-chloro-6-(morpholin-4-yl)pyrimidine (white solid, 60%).

¹H NMR (300 MHz, CDCl₃), δ (ppm): 6.20 (s, 1H); 4.26 (t, *J* = 6.6 Hz, 2H); 3.78 - 3.70 (m, 4H); 3.66 -3.56 (m, 4H); 1.80 -1.68 (m, 2H); 1.54 - 1.40 (m, 2H); and 0.96 (t, *J* = 6.9, 3H).

MS (ESI): m/z 272.1 (M+H).

To a solution of 2-n-butoxy-4-chloro-6-(morpholin-4-yl)pyrimidine (1.38 g, 5.1 mmol) in dioxane (50ml), anhydrous hydrazine (1.6 mL, 50 mmol) was added. The obtained reaction mixture was heated to 95°C, and stirred for 12 h under N₂. After cooling to room temperature, the reaction mixture was quenched with ice-brine and extracted with EtOAc (200 mL). The organic extract was washed with brine, water, and dried over Na₂SO₄. The solvent was removed under reduced pressure. The crude residue was recrystallized from methanol to obtain 2-n-butoxy-4-hydrazino-6-(morpholin-4-yl)pyrimidine as white crystals (1.10 g, 81%).

¹H NMR (300 MHz, CDCl₃), δ (ppm): 5.89 (br s, 1H), 5.49 (s, 1H), 4.26 (t, *J* = 6.6, 2H), 3.84-3.78 (m, 6H), 3.62 -3.47 (m, 4H), 1.82 - 1.67 (m, 2H), 1.55 - 1.42 (m, 2H), and 0.96 (t, *J* = 6.9, 3H);

MS (ESI): m/z 268.2 (M+H).

To a solution of 2-n-butoxy-4-hydrazino-6-(morpholin-4-yl)pyrimidine (200 mg, 0.748 mmol) in MeOH (20 mL), indole-3-carboxaldehyde (108.6 mg, 0.748 mmol) and acetic acid (a drop) were added sequentially. The obtained reaction mixture was stirred at room temperature for 12 h. A white precipitate was formed, collected, and washed with 2 mL methanol to give 200g of Compound 2 (68%).

¹H NMR (300 MHz, CDCl₃), δ (ppm): 8.36 (br s, 1H), 8.30 (dd, *J* = 6.6, 1.8, 1H), 8.05 (s, 1H), 8.00 (s, 1H), 7.44- 7.40 (m, 2H), 7.33 - 7.24 (m, 2H), 6.13 (s, 1H), 4.26 (t, 2H, *J* = 6.6), 3.84 - 3.78 (m, 4H), 3.70 -3.64 (m, 4H), 1.80 -1.70 (m, 2H), 1.54 -1.42 (m, 2H), and 0.96 (t, *J* = 6.9, 3H);

MS (ESI): m/z 395.2 (M+H).

### Example 3. Preparation of Compound 3: N-(2-(4-hydroxybutyl)-6-morpholin-4-yl-pyrimidin-4-yl)-N'-(1H-indol-3-ylmethylene)-hydrazine

A mixture of 4-ethoxy-4-oxo-butylzinc bromide (50 mL 0.5M in THF, 25 mmol), 2,4-dichloro-6-morpholinopyrimidine (4.68 g, 20.0 mmol) and *trans-*benzyl(chloro)bis(triphenylphosphine)palladium(II) (0.15 g, 0.2 mmol) in THF (total volume 80 mL) was stirred at 60°C for 2 days. After routine workup, flash chromatography purification was performed to obtain 4-chloro-2-(4-ethoxy-4-oxobutyl)-6-morpholinopyrimidine as a white solid (2.073 g, 6.60 mmol, 33.0%).

To a solution of 4-chloro-2-(4-ethoxy-4-oxo-butyl)-6-morpholinopyrimidine (1.108 g, 3.54 mmol) in 50 mL THF at -78°C, a diisobutylaluminum hydride (DIBAL) solution (4.72 mL, 1.5 M in toluene, 7.08 mmol) was slowly added. After addition, the obtained reaction mixture was warmed up slowly to 0°C and kept at 0°C for 10 min. After routine workup, flash chromatography was performed to obtain 4-chloro-2-(4-hydroxybutyl)-6-morpholinopyrimidine (0.76 g, 2.80 mmol, 79%) as light yellow solid.

¹H NMR (300 MHz, CDCl₃), δ (ppm): 6.33 (s, 1H), 3.76-3.79 (m, 4H); 3.61-3.68 (m, 6H); 2.76 (t, *J* = 7.8 Hz, 2H); 1.81-1.91 (m, 2H); and 1.60-1.74 (m, 3H).

MS (ESI): m/z 370.2 (M+H).

Following the typical procedure, 4-chloro-2-(4-hydroxybutyl)-6-morpholinopyrimidine (0.542 g, 2.00 mmol, 1.00 equiv.) was reacted with hydrazine and indole-3-carboxaldehyde to give Compound 3 as an off-white solid (0.75 g, 1.90 mmol, 95%).

¹H NMR (300 MHz, DMSO-d₆), δ (ppm): 11.47 (s, 1H); 10.64 (s, 1H); 8.25 (s, 1H); 8.18 (d, *J* = 6.6 Hz, 1H); 7.71 (s, 1H); 7.43 (d, *J* = 8.4 Hz, 1H); 7.17-7.20 (m, 2H); 6.16 (s, 1H), 4.37 (t, *J* = 4.8 Hz, 1H); 3.72 (br s, 4H); 3.55 (br s, 4H); 3.41-3.45 (m, 2H); 2.49-2.54 (m, 2H), 1.66-1.76 (m,2H); and 1.42-1.53 (m,2H).

MS (ESI): m/z 395.1 (M+H).

### Example 4. Preparation of Compound 4: N-[2-(2-[1,3]dioxan-2-yl-ethyl)-6-morpholin-4-yl-pyrimidin-4-yl]-N'-(1H-indol-3-ylmethylene)-hydrazine

Compound 4 was prepared in a similar manner as described in Example 1.

¹H NMR (300 MHz, DMSO-d₆), δ (ppm): 11.46 (s, 1H); 10.64 (s, 1H); 8.25 (s, 1H); 8.18 (d, *J* = 6.6 Hz, 1H); 7.71 (s, 1H); 7.43 (d, *J* = 6.0 Hz, 7.5 Hz, 1H); 7.16-7.19 (m, 2H); 6.15 (s, 1H), 4.58 (t, *J* = 5.1 Hz, 1H); 4.00 (dd, *J* = 11.4 Hz, 4.5 Hz, 2H); 3.64-3.72 (m, 6H); 3.54 (br s, 4H); 2.50-2.59 (m, 2H); 1.80-1.94 (m, 3H), and 1.33 (d, *J* = 9.6 Hz, 1H).

MS (ESI): m/z 437.2 (M+H).

### Example 5. Preparation of Compound 5: N-(1H-indol-3-ylmethylene-N'-[2-(3-methoxy-propyl)-6-morpholin-4-yl-pyrimidin-4-yl]-hydrazine

Following the procedure for the synthesis of N-(2-(4-hydroxybutyl)-6-morpholin-4-yl-pyrimidin-4-yl)-N'-(1H-indol-3-ylmethylene)-hydrazine (Compound 3), 4-chloro-2-(3-hydroxypropyl)-6-morpholinopyrimidine (0.81 g, 3.15 mmol) was synthesized, methylated with sodium hydride (0.48 g, 6.30 mmol) for 10 min, and MeI (0.895 g, 6.30 mmol) for 5 h in 30 mL TTF at 0°C to give 4-chloro-2-(3-methoxypropyl)-6-morpholinopyrimidine as colorless viscous oil (0.792 g, 3.03 mmol, 96%).

¹H NMR (300 MHz, CDCl₃), δ (ppm): 6.32 (s, 1H), 3.75-3.79 (m, 4H); 3.61-3.64 (m, 4H); 3.44 (t, *J =* 6.6 Hz, 2H); 3.34 (s, 3H); 2.78 (t, *J =* 7.8 Hz, 2H); and 2.00-2.09 (m, 2H).

MS (ESI): m/z 262.1 (M+H).

Following the typical procedure, 4-chloro-2-(3-methoxypropyl)-6-morpholinopyrimidine (0.783 g, 3.00 mmol) was treated with hydrazine and indole-3-carboxaldehyde sequentially to yield 0.89 g of Compound 5 (2.26 mmol, 75%).

¹H NMR (300 MHz, DMSO-d₆), δ (ppm): 11.46 (s, 1H); 10.64 (s, 1H); 8.26 (s, 1H); 8.17-8.20 (m, 1H); 7.72 (d, *J* = 2.4 Hz, 1H); 7.43 (dd, *J* = 6.0 Hz, 2.4 Hz, 1H); 7.15-7.21 (m, 2H); 6.16 (s, 1H), 3.70-3.73 (m, 4H); 3.52-3.56 (m, 4H); 3.37 (t, *J* = 6.9 Hz, 2H); 3.23 (s, 3H); 2.50-2.57 (m, 2H), and 1.88-1.97 (m, 2H).

MS (ESI): m/z 395.2 (M+H).

### Example 6. Preparation of Compound 6: 3-{4-[N'-(1H-indol-3-ylmethylene)-hydrazino]-6-morpholin-4-yl-pyrimidin-2-ylsulfanyl}-propan-1-ol

Compound 6 was prepared in a similar manner as described in Example 2.

¹H NMR (300 MHz, DMSO-d₆), δ (ppm): 11.48 (s, 1H); 10.68 (s, 1H); 8.26 (s, 1H); 8.15-8.18 (m, 1H); 7.73 (d, *J* = 2.1 Hz, 1H); 7.42-7.44 (m, 1H); 7.16-7.20 (m, 2H); 6.04 (s, 1H), 4.53 (*t, J =* 5.1 Hz, 1H); 3.65-3.71 (m, 4H); 3.48-3.56 (m, 6H); 3.06 (t, *J =* 7.2 Hz, 2H), and 1.76-1.85 (m, 2H).

MS (ESI): m/z 413.1 (M+H).

### Example 7. Preparation of Compound 7: 3-{2-[N'-(1H-indol-3-ylmethylene)-hydrazino]-6-morpholin-4-yl-pyrimidin-4-ylsulfanyl}-propan-1-ol

Compound 7 was prepared in a similar manner as described in Example 2.

¹H NMR (300 MHz, DMSO-d₆), δ (ppm): 11.34 (s, 1H); 10.48 (s, 1H); 8.45 (d, *J* = 7.8 Hz, 1H); 8.25 (s, 1H); 7.64 (d, *J* = 2.7 Hz, 1H); 7.40 (d, *J* = 8.1 Hz, 1H); 7.05-7.19 (m, 2H); 6.08 (s, 1H), 4.60 (t, *J* = 5.1 Hz, 1H); 3.50-3.68 (m, 10H); 3.20-3.30 (m, 2H); and 1.78-1.86 (m, 2H).

MS (ESI): m/z 413.1 (M+H).

### Example 8. Preparation of Compound 8: N-[2-(2,2-dimethyl-[1,3]dioxolan-4-ylmethoxy)-6-morpholin-4-yl-pyrimidin-4-yl]-N'-(1H-indol-3-ylmethylene)-hydrazine

Compound 8 was prepared in a similar manner as described in Example 2.

¹H NMR (300 MHz, CDCl₃), δ (ppm): 8.38 (br s, 1H); 8.30 (dd, *J* = 7.2, 1.8, 1H), 8.02 (br s, 1H); 8.00 (s, 1H); 7.44 - 7.41 (m, 2H); 7.32 - 7.26 (m, 2H); 6.14 (s, 1H); 4.51-4.42 (m, 2H);, 4.22 - 4.12 (m, 2H); 3.96 - 3.91 (m, 1H); 3.84 - 3.79 (m, 4H); 3.70 - 3.64 (m, 4H); 1.47 (s, 3H); and 1.38(s, 3H).

MS (ESI): m/z 453.2 (M+H).

### Example 9. Preparation of Compound 9: N-{2-[2-(3,4-dimethoxy-phenyl)-ethoxy]-6-morpholin-4-yl-pyrimidin-4-yl}-N'-(1H-indol-3-ylmethylene)-hydrazine

Compound 9 was prepared in a similar manner as described in Example 2.

¹H NMR (300 MHz, CDCl₃), δ (ppm): 8.43 (bs, 1H); 8.30 (d, *J* = 7.5Hz 1H); 8.2 (bs, 1H); 8.02 (d, *J* = 2.7Hz, 1H); 7.46-7.40 (m, 2H); 7.30-7.26 (m, 2H); 6.82 (d, *J* = 1 Hz, 3H); 4.45 (d, *J* = 3.6Hz, 1H); 4.45 (t, *J* = 5.2 Hz, 2H); 3.87 (d, *J* = 3.9Hz, 3H); 3.86 (d, *J* = 3.9Hz, 3H); 3.81 (s, 4H); 3.67(s, 4H); and 3.04 (t, *J* = 5.0 Hz, 2H).

MS (ESI): m/z 503.2 (M+H).

### Example 10. Preparation of Compound 10: N-(1H-indol-3-ylmethylene)-N'-[6-morpholin-4-yl-2-(2-pyridin-2-yl-ethoxy)-pyrimidin-4-yl]-hydrazine

Compound 10 was prepared in a similar manner as described in Example 2.

¹H NMR (300 MHz, CDCl₃ ), δ (ppm): 9.3 (bs, 1H); 8.66 (s, 1H); 8.55-8.53 (m, 1H); 8.28-8.26 (m, 1H); 8.04 (s, 1H); 7.62-7.57 (m, 1H); 7.41-7.10 (m, 6H); 6.08 (s, 1H); 4.64 (t, *J* = 6.6Hz, 2H); 3.76 (s, 4H); 3.62 (s, 4H); and 3.26 (t, *J* = 6.6Hz, 2H).

MS (ESI): m/z 444.2 (M+H).

### Example 11. Preparation of Compound 11: N-(1H-indol-3-ylmethylene)-N'-[6-morpholin-4-yl-2-(3-pyridin-2-yl-propyl)-pyrimidin-4-yl]-hydrazine

Compound 11 was prepared in a similar manner as described in Example 1.

¹H NMR (300 MHz, DMSO-d₆), δ (ppm): 11.47 (s, 1H); 10.65 (s, 1H); 8.50 (d, *J* = 4.5 Hz, 1H); 8.26 (s, 1H); 8.20-8.18 (m, 1H); 7.72-7.68 (m, 2H); 7.45-7.42 (m, 1H); 7.29-7.18 (m, 4H); 6.17(s, 1H); 3.73 (s, 4H); 3.5 (s, 4H); 2.79 (t, *J* = 7.5 Hz, 2H); 2-58-2.51 (m, 2H); and 2.18-2.06 (m, 2H).

MS (ESI): m/z 442.2 (M+H).

### Example 12. Preparation of Compound 12: N-(3-methyl-benzylidene)-N'-[6-morpholin-4-yl-2-(2-pyridin-2-yl-ethoxy)-pyrimidin-4-yl]-hydrazine

Compound 12 was prepared in a similar manner as described in Example 2.

¹H NMR (300 MHz, CDCl₃), δ (ppm): 8.55-8.48 (m, 2H); 7.71 (s, 1H); 7.65-7.55 (m, 1H); 7.49-7.42 (m, 2H); 7.30-7.15 (m, 4H); 6.08 (s, 1H); 4.64 (t, *J* = 6.6 Hz, 2H); 3.81-3.75 (m, 4H); 3.64-3.61 (m, 4H); 3.25 (t, *J* = 7.0 Hz, 2H); and 2.38 (s, 3H).

MS (ESI): m/z 419.2 (M+H).

### Example 13. Preparation of Compound 13: N-(3-ethyl-benzylidene)-N'-[6-morpholin-4-yl-2-(2-pyridin-2-yl-ethoxy)-pyrimidin-4-yl]-hydrazine

Compound 13 was prepared in a similar manner as described in Example 2.

¹H NMR (300 MHz, CDCl₃), δ (ppm): 8.58-8.50 (m, 1H); 8.43 (s, 1H); 7.95 (s, 1H); 7.64-7.58 (m, 2H); 7.30-7.25 (m, 1H); 7.18-7.05 (m, 3H); 6.07(s, 1H); 4.65 (t, *J* = 6.9 Hz, 2H); 3.80-3.76 (m, 4H); 3.64-3.61(m, 4H); 3.26(t, *J* = 6.9 Hz, 2H); 2.40 (q, *J* = 7.6 Hz, 2H); and 1.45 (t, *J* = 7.6 Hz, 3H).

MS (ESI): m/z 433.3 (M+H).

### Example 14. Preparation of Compound 14: N-(3-methyl-benzylidene)-N'-[6-morpholin-4-yl-2-(3-pyridin-2-yl-propyl)-pyrimidin-4-yl]-hydrazine

Compound 14 was prepared in a similar manner as described in Example 1.

¹H NMR (300 MHz, CDCl₃), δ (ppm): 9.6 (bs, 1H); 8.53 (d, *J* = 4.5 Hz, 1H); 7.76 (s, 1H); 7.56 (t, *J* = 6 Hz, 1H); 7.49-7.47 (m, 2H); 7.28 (m, 1H); 7.18-7.06 (m, 3H); 6.26 (s, 1H); 3.81-3.79 (m, 4H); 3.69-3.67 (m, 4H); 2.89 (t, *J* = 7.8Hz, 2H); 2.71 (t, *J* = 7.5 Hz, 2H); 2.39 (s, 3H); and 2.22 (t, *J* = 7.5Hz, 2H).

MS (ESI): m/z 417.2 (M+H).

### Example 15. Preparation of Compound 15: N-[6-morpholin-4-yl-2-(2-pyridin-2-yl-ethoxy)-pyrimidin-4-yl]-N'-(1-m-tolyl-ethylidene)-hydrazine

Compound 15 was prepared in a similar manner as described in Example 2.

¹H NMR (300 MHz, CDCl₃), δ (ppm): 8.56 (bs, 1H), 7.66-7.46 (m, 4H), 7.32-7.26 (m, 2H), 7.16-7.14 (m, 2H), 6.44(s, 1H), 4.69 (t, J = 6.9Hz, 2H), 3.80-3.77 (m, 4H), 3.63-3.60 (m, 4H), 3.31 (t, *J* = 6.9Hz, 2H), 2.39 (s, 3H).

MS (ESI): m/z 433.2 (M+H).

### Example 16. Preparation of Compound 16: N-[1H-indol-3-yl)-ethylidene]-N'-[6-morpholin-4-yl-2-(2-pyridin-2-yl-ethoxy)-pyrimidin-4-yl]-hydrazine

Compound 16 was prepared in a similar manner as described in Example 2.

¹H NMR (300 MHz, CDCl₃), δ (ppm): 9.35 (bs, 1H); 8.54 (dd, *J* = 0.9,4.2 Hz, 1H); 8.33 (d, *J* = 7.5 Hz, 1H); 7.93 (s, 1H); 7.58 (t, *J* = 7.2 Hz, 1H); 7.36-7.33 (m, 2H); 7.27-7.120 (m, 4H); 6.49 (s, 1H); 4.68 (t, *J* = 7.2 Hz, 2H); 3.76-3.73 (m, 4H); 3.60-3-57 (m, 4H); 3.50 (s, 3H); and 3.33-3.28 (t, *J* = 7.0 Hz, 2H).

MS (ESI): m/z 458.2 (M+H).

### Example 17. Preparation of Compound 17: 3-Methyl-benzaldehyde O-[6-morpholin-4-yl-2-(2-pyridin-2-yl-ethoxy)-pyrimidin-4-yl]-oxime

Compound 17 was prepared in a similar manner as described in Example 2.

¹H NMR (300 MHz, CDCl₃), δ (ppm): 8.56-8.53 (m, 1H); 8.45 (s, 1H); 7.62-7.50 (m, 3H); 7.38-7.26 (m, 3H); 7.18-7.10 (m, 1H); 6.17 (s, 1H); 4.68 (t, *J* = 6.9 Hz, 2H); 3.80-3.76 (m, 4H); 3.67-3.64 (m, 4H); 3.29 (t, *J* = 6.9Hz, 2H); and 2.41 (s, 3H).

MS (ESI): m/z 420.1 (M+H).

### Example 18. Preparation of Compound 18: 1H-indole-3-carbaldehyde O-[6-morpholin-4-yl-2-(2-pyridin-2-yl-ethoxy)-pyrimidin-4-yl]-oxime

Compound 18 was prepared in a similar manner as described in Example 2.

¹H NMR (300 MHz, DMSO-d₆), δ (ppm): 11.82 (bs, 1H); 8.81 (s, 1H); 8.50 (d, *J* = 4.5 Hz, 1H); 8.04 (d, J=6.9Hz, 1H); 7.93(s, 1H); 7.72 (t, J = 6.9 Hz,1H); 7.49 (d, *J* = 6.9 Hz, 1H); 7.33 (d, *J* = 7.8Hz, 1H); 7.30-7.18 (m, 3H); 6.22 (s, 1H); 4.57 (t, *J* = 6.3Hz, 2H); 3.67 (s, 4H); 3.56 (s, 4H); and 3.15 (t, J=6.3 Hz, 2H).

MS (ESI): m/z 445.2 (M+H).

### Example 19. Preparation of Compound 19: N-(1H-indol-3-ylmethylene)-N'-{6-morpholin-4-yl-2-[2-(pyridin-3-yloxy)-ethoxy]-pyrimidin-4-yl}-hydrazine

Compound 19 was prepared in a similar manner as described in Example 2.

¹H NMR: (300 MHz, CDCl₃), δ (ppm): 9.20 (br s, 1H); 8.30 (br s, 1H); 8.29 (t, *J* = 3.3 Hz, 1H); 8.18-8.12 (m, 2H); 7.44 -7.41 (m, 2H); 7.26-7.18 (m, 5H); 6.08 (s, 1H); 4.66 (t, *J* = 4.8 Hz, 2H); 4.29 (t, *J* = 5.0 Hz, 2H); 3.80-3.76 (m, 4H); and 3.67-3.62 (m, 4H).

MS (ESI): m/z 460.2 (M+H).

### Example 20. Preparation of Compound 20: N-(3-methyl-benzylidene)-N'-{6-morpholin-4-yl-2-[2-(pyridin-3-yloxy)-ethoxy]-pyrimidin-4-yl}-hydrazine

Compound 20 was prepared in a similar manner as described in Example 2.

¹H NMR (300 MHz, CDCl₃), δ (ppm): 8.55 (s, 1H); 8.34 (br s, 1H); 8.30-8.23 (m, 1H); 7.78 (s, 1H); 7.50-7.47 (m, 2H); 7.32-7.24 (m, 1H); 7.20-7.17 (m, 3H); 6.14 (s, 1H); 4.66 (t, *J* = 5.0 Hz, 2H); 4.35 (t, *J* = 4.8 Hz, 2H); 3.83-3.80 (m, 4H); 3.68-3.65 (m, 4H); and 2.40 (s, 3H).

MS (ESI): m/z 435.2 (M+H).

### Example 21. Preparation of Compound 21: Butyl-{4-[N'-(1H-indol-3-ylmethylene)-hydrazino]-6-moropholin-4-yl-pyrimidin-2-yl}-amine

Compound 21 was prepared in a similar manner as described in Example 2.

¹H NMR (300 MHz, CDCl₃), δ ppm: 8.41 (bs, 1H), 8.33-8.30 (m, 1H), 8.19 (bs, 1H), 7.95 (s, 1H), 7.41-7.37 (m, 2H), 7.29-7.25 (m, 2H), 5.96 (s,1H), 4.65 (t, *J*=4 Hz, 1H), 3.83-3.80 (m, 4H), 3.65-3.62 (m, 4H), 3.36 (dd, *J*=6.3, 13.5 Hz, 2H), 1.60-1.55 (m, 2H), 1.35-1.33 (m, 4H), 0.92-0.87 (m, 3H).

MS (ESI): m/z 408.2 (M+H).

### Example 22. Preparation of Compound 22: N-(3-methyl-benzylidene)-N'-[6-morpholin-4-yl-2-(pyridin-3 yloxy)-pyrimidin-4-yl]-hydrazine

To a solution of 3-hydroxypyridine (950 mg, 10 mmol) in anhydrous THF (50 mL) at 0°C under nitrogen protection was added NaH (60% in oil) (480 mg, 12 mmol). The suspension was stirred for 0.5 h at 0°C, and 2,4,6-trichloropyrimidine (1.84 g, 10 mmol) was added. After the mixture warmed to room temperature and stirred for 2 h, the reaction was quenched by ice brine and extracted with EtOAc (300 mL). The organic phase was washed with brine, dried (Na₂SO₄), filtered, evaporated in *vacuo.* The cure product was purified by flash chromatography on a column of silica gel (EtOAc-hexane, 1:7). The product (1.80g, 7.4mmol) in CH₂Cl₂ (150 mL) at 0°C was added slowly morpholine (2.5g, 28 mmol). The reaction mixture was stirred at 0°C for 1 h and another 1 h at room temperature. The mixture was washed with water. The organic phase was dried (Na₂SO₄), filtered and evaporated in *vacuo* and presented three isomers. The isomers was separated by flash chromatography on a column of silica gel (EtOAc-hexane, 1:7 and 1: 3) to obtain 4-[6-chloro-2-(pyridin-3-yloxy)-pyrimidin-4-yl]-morpholine (320mg, 14.7%).

¹H NMR (300 MHz, CDCl₃), δ (ppm): 8.51 (d, 1H, *J*=2.7 Hz), 8.44 (dd, 1H, J=1.5, J=3.3 Hz), 7.53-7.49 (m, 1H), 7.34-7.3 (m, 1H), 6.25 (s, 1H), 3.71-3.67 (m, 4H), 3.51-3.48 (m, 4H).

MS (ESI): m/z 293.1.

To a solution of 4-[6-chloro-2-(pyridin-3-yloxy)-pyrimidin-4-yl]-morpholine (295mg, 1 mmol) in THF (10 mL) was added anhydrous hydrazine (0,320 ml, 10 mmol) under nitrogen protection. The mixture was heated at 70°C for 15 min. After cooling to room temperature, the reaction mixture was quenched by ice brine and extracted with EtOAc (100 mL). The organic phase was washed with brine (10 mL) and water (10ml x 2), dried (Na₂SO₄), filtered, evaporated, and purified by flash chromatography on a column of silica gel (CH₂Cl₂ and CH₂Cl₂-MeOH, 95:5) and to give [6-morpholin-4-yl-2-(pyridin-3-yloxy)-pyrimidin-4-yl]-hydrazine (180 mg) in 62% yield. M/Z (M+1) 289.2

To a solution of [6-morpholin-4-yl-2-(pyridin-3-yloxy)-pyrimidin-4-yl]-hydrazine (180 mg) (145 mg, 0.5 mmol) and m-tolylaldehyde (72 mg, 0.6 mmol) in MeOH (10 mL) was added acetic acid (1 drop). The reaction mixture was stirred at room temperature for 12 h and a white solid was precipitated. The resulting precipitate was collected by filtration and washed with little amount ofmetanol and to give 125 mg of Compound 22 in 64 % yield.

¹H NMR (300 MHz, CDCl₃), δ (ppm): 8.71 (s, 1H), 8.57 (d, 1H, *J=2.4* Hz), 8.44 (dd, 1H, *J=1.5,* 3.2 Hz), 7.78 (s,1H), 7.56-7.52 (m,1H), 7.46-7.43 (m, 2H), 7.34-7.26 (m, 2H), 7.17 (d, 1H, *J*=8.1 Hz), 6.17 (s, 1H), 3.76-3.73 (m, 4H), 3.57-3.54 (m, 4H), 2.38 (s, 3H).

MS (ESI): m/z 391.2.

### Example 23. Preparation of Compound 23: N-(3-methylbenzlidene)-N'-(5-methyl-6-morpholin-4-yl-2-phenylpyrimidin-4-yl)hydrazine

Benzamidine hydrochloride (7.06 g, 0.045 mol) and dimethyl methylmalonate (6.0 g, 0.041 mol) were dissolved in methanol (100 mL). Sodium methoxide (21.5 mL, 0.099 mol, 25 wt % solution in methanol) was added and the solution was stirred at room temperature for 18 h. The volume of solvent was redcued to approximately 50 mL under reduced pressure, then poured onto ice water. This solution was neutralized with HOAc which produced a white precipitate. This precipitate was collected and dried to produce a white solid (6.1 g, 74 %).

¹H NMR (DMSO-d₆), δ (ppm):1.68 (s, 3H), 7.70-7.87 (m, 3H), 8.21 (d, J=8.4 Hz).

MS (ESI): m/z 203.1 (M+H)⁺

5-Methyl-2-phenyl-pyrimidine-4,6-diol (3.3 g, 0.016 mol) and POCl₃ were heated to 60°C for 3 hrs. The solution was allowed to cool to room temperature then poured onto ice. The resultant white precipitate was filtered and dried to produce the desired compound as a white solid (810 mg, 21 %).

¹H NMR (DMSO-d₆), δ (ppm):2.40 (s, 3H), 7.51-7.56 (m, 3H), 8.23 (d, 8.4 Hz).

MS (ESI): m/z 239.1 (M+H)⁺

4,6-Dichloro-5-methyl-2-phenylpyrimidine (2.5 g, 0.010 mol) and morpholine (2.93 g, 0.031 mol) were dissolved in THF (50 mL) and heated to reflux for 3 hrs. The solution was allowed to cool then EtOAc (100 mL) and water (100 mL) were added. The EtOAc layer was washed with water (3x100 mL), dried over MgSO₄, filtered and solvent was removed under reduced pressure. The resultant solid was used without further purification (2.66 g, 92 %).

MS (ESI): m/z 298.1 (M+H)⁺

4-(6-Chloro-5-methyl-2-phenylpyrimidin-4-yl)morpholine (439 mg, 1.51 mmol) was dissolved in THF (50 mL). Hydrazine (0.25 mL, 7.96 mmol) was added and the solution was heated to reflux for 18 hrs. The reaction was allowed to cool the solvent was removed under reduced pressure. EtOAc (100 mL) and water (100 mL) were added. The EtOAc layer was washed with water (3x100 mL), dried over MgSO₄, filtered and solvent was removed under reduced pressure to produce a white solid (374 mg). This solid was redissolved in THF (50 mL) and m-tolualdehyde (157 mg, 1.31 mmol) was added. The solution was heated to reflux for 4 hrs then allowed to cool. Solvent was removed under reduced pressure then EtOAc (100 mL) and water (100 mL) were added. The EtOAc layer was washed with water (3x100 mL), dried over MgSO₄, filtered and solvent was removed under reduced pressure. The crude product was purified by silcagel column chromatography, eluting with 25 % EtOAc/hexane to produce the pure desired product as a yellow solid (313 mg, 53 %).

¹H NMR (DMSO-d₆), δ (ppm) 2.26 (s, 3H), 2.36 (s, 3H), 3.35 (m, 4H), 3.75-3.78 (m, 4H), 7.20 (d, J=6.9 Hz), 7.33 (t, J=6.9 Hz), 7.47-7.52 (m, 5H), 8.19 (s, 1H), 8.35-8.38 (m, 2H), 10.60 (s, 1H).

MS (ESI): m/z 388.3 (M+H)⁺

### Example 24. Preparation of compound 24: N-(3-methyl-benzylidenel-N'-(2-phenyl-6-thiomorpholin-4-yl-pyrimidin-4-yl)-hydrazine

Compound 24 was prepared in a similar manner as described in Example 23.

¹H-NMR (DMSO-d₆), δ 2.36 (s, 3H), 2.76 (s, 4H), 4.07 (s, 4H), 6.36 (s, 1H), 7.19 (d, J=8.1 Hz), 7.32 (t, *J*=8.1 Hz), 7.47-7.57 (m, 5H), 8.09 (s, 1H), 8.30-8.31 (m, 1H), 11.02 (s, 1H).

MS (ESI): m/z 389.1.

### Example 25. Preparation of (2,3-dimethyl-1H-indole-5-yl)-{6-morpholin-4-yl-2-[2-pyridin-3-yloxy)-ethoxy]-pyrimidin-4-yl}-amine (reference example)

To a solution of 2-(pyridin-3-yloxy)-ethanol (3.48 g, 25 mmol) in 40 mL of anhydrous THF at room temperature under N₂, 2,4,6-trichloro-pyrimidine (4.56 g, 25 mmol) was added followed by portionwise addition ofNaH (60% suspension in oil, 1.1 g, 27.5 mmol). After 30 min of stirring the reaction was quenched with water, the water layer was extracted with EtOAc, and the combined organic solutions were washed with brine and dried over MgSO₄.

Purification using flash chromatography (silica; dichloromethane/acetone/methanol : 3/1/0.1) afforded a mixture of 4,6-dichloro-2- and 2,6-dichloro-4- [2-(pyridin-3-yloxy)-ethoxy]-pyrimidines (3.72 g, 52%), (NMR ratio 1:1.2) as an oil.

To a solution of the above mixture (3.72 g, 13 mmol) in 20 mL of 1,4-dioxane was added DIPEA (2.49 mL, 14.3 mmol), followed by 2,3-dimethyl-5-amino-indole (2.08 g, 13 mmol) and the mixture was refluxed for 1 hour. Solvent was removed under reduced pressure and the reaction mixture was separated using column chromatography (silica; dichloromethane/acetone/methanol: 3/1/0.1) to afford {6-chloro-2-[2-(pyridin-3-yloxy)-ethoxy]-pyrimidin-4-yl}-amine (2.07 g, 39%). A mixture of {4-chloro-6-[2-(pyridin-3-yloxy)-ethoxy]-pyrimidin-4-yl}-amine and {2-chloro-6-[2-(pyridin-3-yloxy)-ethoxy]-pyrimidin-4-yl}-amine (2.5 g, 47%) was also obtained and used in another reaction.

A solution of {6-chloro-2-[2-(pyridin-3-yloxy)-ethoxy]-pyrimidin-4-yl}-amine (2.07 g, 5.05 mmol) and morpholine (1.32 mL, 15.15 mmol) in 1,4-dioxane was heated at 110 °C for 24 hours. Solvent was removed under reduced pressure and the reaction mixture was purified using flash chromatography (silica; dichloromethane/acetone/methanol: 3/1/0.1) to afford the title compound (2 g, 86%) as a colorless solid.

¹H NMR (300 MHz, CDCl₃), δ (ppm): 8.34 (br s, 1H), 8.23 (dd, 1H, J= 3.6, 2.1), 7.96 (brs, 1H), 7.34-7.21 (m, 4H), 6.98(dd, 1H, *J*= 8.4, 1.8 Hz), 6.60 (brs, 1H), 5.36 (s, 1H), 4.65 (t, 2H, *J*=5.1 Hz), 4.34 (t, 2H, *J*=5.1 Hz), 3.66 (m, 4H), 3.42 (m, 4H), 2.37(s, 3H), and 2.20 (s, 3H).

MS (ESI): m/z 461.5 (M+H).

### Example 26. Preparation of Compound 25: 3-{4-[N'-(3-methyl-benzylidene)-hydrazino]-6-morpholin-4-yl-pyrimidin-2-yl}-propionic acid ethyl ester

Compound 25 was prepared in a similar manner as described in Example 1.

¹H NMR (300 MHz, CDCl₃), δ (ppm): 8.22 (s, 1H); 7.69 (s, 1H); 8.07 (s, 1H); 7.47 (m, 2H); 7.28 (t, *J* = 7.5 Hz, 1H); 7.17 (d, *J* = 7.5 Hz, 1H); 6.23(s, 1H); 4.13 (q, *J* = 7.2 Hz, 2H); 3.78-3.81 (m, 4H); 3.62-3.65 (m, 4H); 2.98 (t, *J* = 7.2 Hz, *2H); 2.77 (t, *J* = 7.2 Hz, 2H); 2.39 (s, 3H); and 1.24 (t, *J* = 7.2 Hz, 3H).

MS (ESI): m/z 398.2 (M+H).

### Example 27. Preparation of Compound 26: N-(3-Methyl-benzylidene)-N'-{6-morpholin-4-yl-2-[2-(1-oxy-pyridin-2-yl)-ethoxy]-pyrimidin-4-yl}-hydrazine

To a solution of 4-[6-chloro-2-(2-pyridin-2-yl-ethoxy)-pyrimidin-4-yl]-morpholine (1.61 g, 5.0 mmol) in CH₂Cl₂ (40 ml) was added methanol (10 ml) followed by the addition of MCPBA (70%, 1.43 g, 5.8 mmol) in one portion. The reaction mixture was stirred overnight at room temperature, affording a clear solution. The solution was cast into saturated aqueous NaHCO₃ (35 mL) then the organic phase was separated, washed with 10% aqueous Na₂S₂O₃ (40 mL) and brine (40 mL), and dried (Na₂SO₄), filtered and evaporated *in vacuo* to give a pure product, 4-{6-chloro-2-[2-(1-oxy-pyridin-2-yl)-ethoxy]-pyimidin-4-yl}-morpholine as a white solid (1.46 g, 86.7%).

¹H-NMR (CDCl₃) (ppm): *J* (Hz): 8.25-8.23 (m, 1H); 7.41-7.7.38 (m, 1H); 7.20-7.16 (m, 2H); 6.14 (s, 1H); 4.71 (t, *J*=6.0, 2H); 3.77-3.73 (m, 4H); 3.63-3.55 (m, 4H); and 3.40 (t, *J*=6.0, 2H).

Anhydrous hydrazine (0.640 ml, 20 mmol) was added to a solution of 4-{6-chloro-2-[2-(1-oxy-pyridin-2-yl)-ethoxy]-pyrimidin-4-yl})-morpholine (1.35 g, 4.0 mmol) in dioxane (15 ml) under nitrogen protection. The obtained mixture was heated at 95-100°C for 2 h. After it was cooled down, the solvent was evaporated in *vacuo* until the white solid began to precipitate (to a half of the original volume), and then H₂O (15 ml) was added. The resulting precipitate was collected by filtration and washed with water (until the pH was neutral). {6-Morpholin-4-yl-2-[2-(1-oxy-pyridin-2-yl)-ethoxy]-pyrimidin-4-yl}-hydrazine (1.02 g) has been obtained in 76.7% yield.

¹H-NMR (DMSO-d₆) (ppm); *J* (Hz): 8.25 (bs, 1H); 7.66(s, 1H); 7.44-7.41 (m, 1H); 7.33-7.25 (m, 2H); 5.59 (s, 1H; 4.46 (t, *J*=6.0, 2H); 3.64-3.61 (m, 4H); 3.41-3.38 (m, 4); and 3.17 (t, *J=6.,* 2H).

To a solution of {6-morpholin-4-yl-2-[2-(1-oxy-pyridin-2-yl)-ethoxy]-pyrimidin-4-yl}-hydrazine (820 mg, 2.46 mmol) and m-tolualdehyde (97%, 320 mg, 2.58 mmol) in methanol (7 mL) acetic acid (2 drops) was added. The reaction mixture was heated under reflux for 15 min. Upon cooling to room temperature, a precipitate has been formed, and the solid was collected by filtration, washed with little amount of methanol and Et₂O, and dried to afford 950 mg (89%) of N-(3-methyl-benzylidene)-N'- {6-morpholin-4-yl-2-[2-(1-oxy-pyridin-2-yl)-ethoxy]-pyrimidin-4-yl}-hydrazine as a white solid (m.p. 187-188°C).

¹H NMR (300 MHz, CDCl₃), δ (ppm): 10.86 (s, 1H); 8.28-8.26 (m, 1H); 7.98 (s, 1H); 7.50-7.43 (m, 3H); 7.33-7.26 (m, 3H); 7.17 (d, *J*=7.8 Hz, 1H); 6.05 (s, 1H); 4.53 (t, *J* =6.3 Hz, 2H); 3.68-3.64 (m, 4H); 3.54-3.50 (m, 4H); 3.21 (t, *J* =6.3, 2H); and 2.33 (s, 3H).

ESMS calcd. for C₂₃H₆N₆O₃: 434.21; Found: 457.2 (M+Na)⁺.

### Example 28. Preparation of Compound 27: 1-(2-{4-[N'-(3-methyl-benzylidene)-hydrazino]-6-moxpholin-4-yl-pyrimidin-2-yloxy}-ethyl)-1H-pyridin-2-one

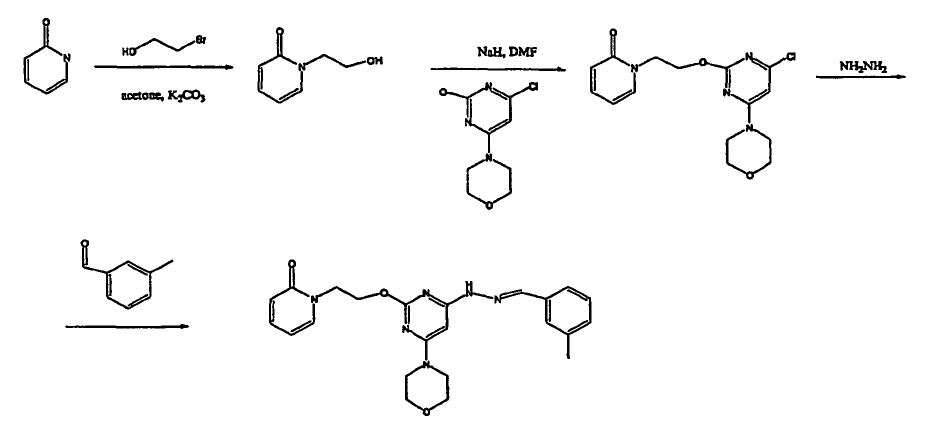

As shown in the scheme above, 1-(hydroxy-ethyl)-1*H*-pyzidin-2-one (1.5 g, 10.7 mmol) was coupled with 4-(2,6-dichloropyrimidin-4-yl)-morpholine in the presence of sodium hydride in DMF. After addition of water, the precipitate was filtered out, washed with water, and dried to afford almost a desired regioisomer (1.7 g, 47%). The obtained regioisomer was refluxed with 3.5 equivalents of hydrazine in dioxane. Water was added to the reaction mixture, and a precipitate was formed. The precipitate was collected by filtration, washed 3 times with water, and dried to give a hydrazine derivative (1.7 g, 85%). Condensation with m-tolyl aldehyde afforded the title compound (2.1 g, 95%).

¹H NMR (DMSO-d₆): δ 10.90 (s, 1H), 7.98 (s, 1H), 7.62 (dd, *J* = 6.8, 2.1Hz, 1H, 7.49 (d, *J* = 7.5 Hz, 1H), 7.48 (s, 1H), 7.41 (td, *J* = 7.8, 2.1 Hz, 1H), 7.29 (t, *J* = 7.5 Hz, 1H), 7.17 (d, *J* = 7.8 Hz, 1H), 6.39 (d, *J* = 9.3 Hz, 1H), 6.20 (t, *J* = 6.2 Hz, 1H), 6.05 (s, 1H), 4.43 (t, *J* = 5.1 Hz, 2H),4.22 (t, *J*= 5.2 Hz, 2H), 3.66 (m, 4H), 3.52 (m, 4H), 2.34 (s, 3H).

ESMS calcd.for C₂₃H₂₆N₆O₃: 434.21; Found: 457.2 (M+23)⁺.

### Example 29. Preparation of Compound 28: N-(3-iodo-benzylidene)-N'-[6-morpholin-4-yl-2-(2-pyridin-2-yl-ethoxy)-pyrimidin-4-yl]-hydrazine

Compound 28 was prepared in a similar manner as described in Example 28.

¹H NMR (DMSO-d₆): δ 10.97 (s, 1H), 8.51 (d, *J* = 4.5 Hz, 1H), 8.00 (s, 1H), 7.95 (s, 1H), 7.78-7.70 (m, 3H), 7.34 (d, *J* = 7.8 Hz, 1H), 7.26-7.18 (m, 2H), 6.08 (s, 1H), 4.55 (t, *J* = 6.6 Hz, 2H), 3.66 (m, 4H), 3.53 (m, 4H), 3.14 (t, *J* = 6.6 Hz, 2H).

ESMS calcd.for C₂₂H₂₃IN₆O₂: 530.09; Found: 531.1 (M+1)⁺.

### Example 30. Preparation of Compound 29: N-(3-fluoro-benzylidene)-N'-[6-morpholin-4-yl-2-(2-pyridin-2-yl-ethoxy)-pyrimidin-4-yl]-hydrazine

Compound 29 was prepared in a similar manner as described in Example 28.

¹H NMR (DMSO-d₆): δ 10.98 (s, 1H), 8.51 (d, *J* = 3.9 Hz, 1H), 8.01 (s, 1H), 7.72 (td, *J =* 7.6, 1.8 Hz, 1H), 7.57 (brd, *J* = 9.9 Hz, 1H), 7.51-7.40 (m, 2H), 7.33 (d, *J* =7.2 Hz, 1H), 7.24 (dd, *J* = 7.6, 5.2 Hz, 1H), 7.20 (brt, *J* = 7.8 Hz, 1H); 6.11 (s, 1H), 4.54 (t, *J* = 6.8 Hz, 2H), 3.65 (m, 4H), 3.54 (m, 4H), 3.14 (t, *J* = 6.7 Hz, 2H).

ESMS calcd.for C₂₂H₂₃FN₆O₂: 422.19; Found: 445.2 (M+23)⁺.

### Example 31, Preparation of Compound 30: N-(3-chloro-benzylidene)-N'-[6-morpholin-4-yl-2-(2-pyridin-2-yl-ethoxy)-pyrimidin-4-yl]-hydrazine

Compound 30 was prepared in a similar manner as described in Example 28.

¹H NMR (DMSO-d₆): δ 11.00 (s, 1H), 8.51 (d, *J* = 4.5 Hz, 1H), 8.00 (s, 1H), 7.74-7.70 (m, 2H), 7.65 (d, *J* = 6.6 Hz, 1H), 7.45-7.41 (m, 2H), 7.33 (d, *J* = 7.8 Hz, 1H), 7.24 (dd, *J =* 7.8, 4.8 Hz, 1H), 6.09 (s, 1H), 4.54 (t, *J* = 6.6 Hz, 2H), 3.66 (m, 4H), 3.54 (m, 4H), 3.14 (t, *J* = 6.6 Hz, 2H).

ESMS calcd.for C₂₂H₂₃ClN₆O₂: 438.16; Found: 461.2 (M+23)⁺.

### Example 32. Preparation of compound 31: N-(3-bromo-benzylidene)-N'-[6-morpholin-4-yl-2-(2-pyridin-2-yl-ethoxy)-pyrimidin-4yl]-hydrazine

Compound 31 was prepared in a similar manner as described in Example 28.

¹H NMR (DMSO-d₆): δ 10.99 (s, 1H), 8.51 (d, *J =* 4.2 Hz, 1H), 7.98 (s, 1H), 7.86 (s, 1H), 7.72 (t, *J* = 8.5 Hz, 1H), 7.71 (d, *J*=8.1 Hz, 1H), 7.54 (d, *J* = 7.5 Hz, 1H), 7.38-7.32 (m, 2H), 7.24 (dd, *J* = 7.2, 4.8 Hz, 1H), 6.09 (s, 1H), 4.54 (t, *J* = 6.6 Hz, 2H), 3.66 (m, 4H), 3.53 (m, 4H), 3.14 (t, *J* = 6.6 Hz, 2H).

ESMS calcd.for C₂₂H₂₃BrN₆O₂: 482.11; Found: 505.10 (M+23)⁺.

### Example 33. Preparation of Compound 32: 3-{[6-Morpholin-4-yl-2-(2-pyridin-2-yl-ethoxy)-pyrimidin-4-yl]-hydrazonomethyl}-benzoic acid methyl ester

Compound 32 was prepared in a similar manner as described in Example 28.

¹H NMR (DMSO-d₆): δ 11.00 (s, 1H), 8.51 (d, *J* = 5.4 Hz, 1H), 8.12 (s, 1H), 8.10 (s, 1H), 8.06 (d, *J* = 8.1 Hz, 1H), 7.93 (d, *J* = 6.6 Hz, 1H), 7.73 (t, *J* = 7.6 Hz, 1H), 7.57 (t, *J =* 8.0 Hz, 1H), 7.34 (d, *J* = 7.8 Hz, 1H), 7.24 (dd, *J* = 6.0,4.5 Hz, 1H), 6.07 (s, 1H), 4.55 (t, *J* = 6.4 Hz, 2H), 3.88 (s, 3H), 3.68 (m, 4H), 3.53 (m, 4H), 3.15 (t, *J* = 6.6 Hz, 2H).

ESMS calcd.for C₂₄H₂₆N₆O₄: 462.20; Found: 463.3 (M+1)⁺.

### Example 34. Preparation of Compound 33: 1-(2-{4-[N'-(3-iodo-benzylidene)-hydrazino]-6-morpholin-4-yl-pyrimidin-2-yloxy}-ethyl)-1H-pyridin-2-one

Compound 33 was prepared in a similar manner as described in Example 28.

¹H NMR (DMSO-d₆): δ 11.02 (s, 1H), 8.00 (s, 1H), 7.93 (s, 1H), 7.75-7.69 (m, 2H), 7.61 (dd, *J* = 7.0, 1.8 Hz, 1H), 7.41 (td, *J* = 7.9, 2.1 Hz, 1H), 7.20 (t, *J* = 8.0 Hz, 1H), 6.38 (d, *J* = 8.4 Hz, 1H), 6.19 (t, *J* = 6.7 Hz, 1H), 6.06 (s, 1H), 4.43 (t, *J* = 5.3 Hz, 2H), 4.22 (t, *J* = 5.3 Hz, 2H), 3.66 (m, 4H), 3.53 (m, 4H), 3.14 (t, *J* = 6.6 Hz, 2H).

ESMS calcd.for C₂₂H₂₃IN₆O₃: 546.09; Found: 569.2 (M+23)⁺.

### Example 35. Preparation of Compound 34: 3-{[6-Morpholin-4-yl-2-(2-pyridin-2-yl-ethoxy)-pyrimidin-4-yl]-hydrazonomethyl}-benzoic acid N-methyl amide

Compound 34 was prepared in a similar manner as described in Example 28.

¹H NMR (DMSO-d₆): δ 11.00 (s, 1H), 8.6 (s, 1H), 8.41 (d, *J* = 5.4 Hz, 1H), 8.12 (s, 1H), 8.11 (s, 1H), 8.0 (d, *J* = 8.1 Hz, 1H), 7.83 (d, *J* = 6.8 Hz, 1H), 7.73 (t, *J* = 7.2 Hz, 1H), 7.57 (t, *J* = 8.0 Hz, 1H), 7.34 (d, *J*= 7.8 Hz, 1H), 7.34 (dd, *J* = 6.0, 4.5 Hz, 1H), 6.07 (s, 1H), 4.55 (t, *J* = 6.4 Hz, 2H), 3.5-3.0 (m, 7H).

ESMS calcd for C₂₄H₂₇N₇O₃: 461.2; Found: 485.1 (M+Na)⁺.

### Example 36. Preparation of Compound 35: (3-{[6-Morpholin-4-yl-2-(2-pyridin-2-yl-ethoxy)-pyrimidin-4-yl]-hydrazonomethyl}-pheny)-methanol

Compound 35 was prepared in a similar manner as described in Example 28.

¹H NMR (DMSO-d₆): δ 10.86 (s, 1H), 8.51 (d, *J* = 3.9 Hz, 1H), 8.03 (s, 1H), 7.73 (td, *J* = 7.8 and 1.8 Hz, 1H), 7.39 (m, 2H), 7.39-7.32 (m, 3H), 7.24 (dd, *J* = 6.3 and 4.8 Hz, 1H), 6.06 (s, 1H), 5.25 (t, *J* = 5.7 Hz, 1H), 4.54 (t, *J* = 6.8 Hz, 2H); 4.53 (d, *J* = 6.5 Hz, 2H), 3.66 (m, 4H), 3.53 (m, 4H), 3.14 (t, *J* = 6.9 Hz, 2H).

ESMS, calcd.for C₂₃H₂₆N₆O₃: 434.49; Found: 435.2 (M+1)⁺.
Compounds 36-39 were prepared by the following method.

4-Carbamimidoyl-butiric acid ethyl ester hydrochloride was prepared following a procedure starting from 4-cyanobutyrate (6.49 g, 43.9 mmol) and coupled with diethyl malonate in the presence of sodium ethylate to afford the desired dihydroxypyrimidine (1.27g, 15%). Treatment of the dihydroxypyrimidine with phosphorus oxychloride gave a dichloro-derivative (0.88 g, 60%), which was converted into a morpholine derivative (0.89 g, 85%) after reacting with DIPEA and morpholine in THF. The dichloro-derivative was refluxed in dioxane with 4 equivalents of hydrazine to afford a hydrazine derivative (0.52 g, 59%) that was condensed with *m-*tolyl aldehyde to obtain a hydrazone (0.61 g, 88%). The hydrazone was hydrolyzed with KOH in methanol to yield an acid: 4-{4-[*N'*-(3-methyl-benzylidene)-hydrazino]-6-morpholin-4-yl-pyrimidin-2-yl}-butyric acid (0.47 g, 82%).

To a solution of the acid, EDC, DMAP, and an appropriate amine in DMF were added. The obtained reaction mixture was stirred overnight at room temperature, and was distributed between dichloromethane and water layers. The dichloromethane layer was washed two times with water, brine, and dried. The obtained amide (70-80% yield) was isolated by column chromatography.

### Example 37. Preparation of Compound 36: N,N-diethyl-4-{4-[N"-(3-methyl-benzylidene)-hydrazino]-6-morpholin-4-yl-pyrimidin-2-yl}-butyramide

¹H NMR (CDCl₃): δ 8.38 (brs, 1H), 7.71 (s, 1H), 7.47 (m, 2H), 7.31-7.26 (m, 2H), 7.17 (d, *J* = 7.5 Hz, 1H), 6.24 (s, 1H), 3.78 (m, 4H), 3.66 (m, 4H), 3.37 (q, *J* = 7.2 Hz, 2H), 3.30 (q, *J* = 7.2 Hz, 2H), 2.67 (t, *J* = 7.4 Hz, 2H), 2.39 (m, 4H), 2.13 (qv, *J* = 7.4 Hz, 2H), 1.13 (t, *J* = 7.4 Hz, 3H), 1.11 (t, *J* = 7.4 Hz, 3H).

ESMS calcd.for C₂₄H₃₄N₆O₂: 438.27; Found: 439.30 (M+1)⁺.

### Example 38 Preparation of Compound 37: 4-{4-[N'-(3-methyl-benzylidene)-hydrazino]-6-morpholin-4-yl-pyrimidin-2-yl}-1-(4-methyl-piperazin-1-yl)-butan-1-one

¹H NMR (CDCl₃): δ 8.36 (brs, 1H), 7.71 (s, 1H), 7.46 (m, 2H), 7.31-7.26 (m, 2H), 7.17 (d, *J* = 7.8 Hz, 1H), 6.25 (s, 1H), 3.80 (m, 4H), 3.65 (m, 6H), 3.46 (t,*J* = 4.9 Hz, 2H), 2.67 (t, *J* = 7.4 Hz, 2H), 2.42-2.34 (m, 8H), 2.30 (s, 3H), 2.11 (qv, *J* = 7.5 Hz, 2H).

ESMS calcd.for C₂₅H₃₅N₇O₂: 465.29; Found: 466.30 (M+1)⁺.

### Example 39. Preparation of Compound 38: 4-{4-[N'-(3-methyl-benzylidene)-hydrazino]-6-morpholin-4-yl-pyrimidin-2-yl}-N-pyridin-4-ylmethyl-butyramide

¹H NMR (CDCl₃): δ 8.59 (brs, 1H), 7.92 (s, 1H), 7.60 (m, 2H), 7.37 (m, 2H), 7.22-7.11 (m, 4H), 7.00 (m, 1H), 6.15 (s, 1H), 4.36 (d, *J* = 5.7 Hz, 2H), 3.68 (m, 4H), 3.53 (m, 4H), 2.62 (t, *J* = 7.4 Hz, 2H), 2.31 (s, 3H), 2.25 (t, *J* = 6.9 Hz, 2H), 2.05 (qv, *J* = 6.8Hz, 2H).

ESMS calcd.for C₂₆H₃₁N₇O₂: 473.25; Found: 474.30 (M+1)⁺.

### Example 40. Preparation of Compound 39: 4-{4-[N'-(3-methyl-benzylidene)-hydrazino]-6-morpholin-4-yl-pyrimidin-2-yl}-N-pyridin-4-yl-butyramide

¹H NMR (CDCl₃): δ 9.43 (s, 1H), 8.68 (brs, 1H), 8.43 (d, *J* = 4.8 Hz, 2H), 7.75 (s, 1H), 7.51 (d, *J* = 5.4 Hz, 2H), 7.44 (m, 2H), 7.27 (t, *J* = 7.2 Hz, 1H), 7.16 (d, *J* = 6.9 Hz, 1H), 6.23 (s, 1H), 3.77 (m, 4H), 3.64 (m, 4H), 2.72 (t, *J* = 6.9Hz, 2H), 2.46 (t, *J* = 6.9 Hz, 2H), 2:37 (s, 3H), 2.15 (qv, *J* = 6.9 Hz, 2H).

ESMS calcd.for C₂₅H₂₉N₇O₂: 459.24; Found: 460.30 (M+1)⁺.

### Example 41. In vitro assays

*Reagents. Staphylococcus aureus* Cowan I (SAC) was obtained from Calbiochem (La Jolla, CA), and lipopolysaccharide (LPS, *Serratia marscencens*) was obtained from Sigma (St. Louis, MO). Human and mouse recombinant IFNγ were purchased from Boehringer Mannheim (Mannheim, Germany) and Pharmingen (San Diego, CA), respectively.

*Human In Vitro Assay.* Human PBMC were isolated by centrifugation using Ficoll-Paque (Pharmacia Biotech, Uppsala, Sweden) and prepared in RPMI medium supplemented with 10% fetal calf serum (FCS), 100 U/mL penicillin, and 100 µg/mL streptomycin. PBMC were plated in wells of a 96-well plate at a concentration of 5x 10⁵ cells/well, and primed by adding IFNγ (30 U/mL) for 22 h and stimulated by adding LPS (1 µg/mL), or by adding IFNγ (100 U/mL) and then stimulated by adding SAC (0.01%). A test pyrimidine compound was dissolved in DMSO, and added to wells of the 96-well plate. The final DMSO concentration was adjusted to 0.25% in all cultures, including the compound-free control. Human THP-1 cells were plated in wells, primed by adding IFNγ (100 U/mL) for 22 h and stimulated by adding SAC (0.025%) in the presence of different concentrations of the pyrimidine compound. Cell-free supernatants were taken 18 h later for measurement of cytokines. Cell viability was assessed using the bioreduction of MTS. Cell survival was estimated by determining the ratio of the absorbance in compound-treated groups versus compound-free control.

The supernatant was assayed for the amount of IL-12p40, IL-12p70, or IL-10 by using a sandwich ELISA with anti-human antibodies, i.e., a Human IL-12 p40 ELISA kit from R&D Systems (Berkeley, CA), and a Human IL-12 p70 or IL-10 ELISA kit from Endogen (Cambridge, MA). Assays were based on the manufacturer's instructions.

*Murine In Vitro Assay.* Balb/c mice (Taconic, Germantown, NY) were immunized with *Mycobacterium tuberculosis* H37Ra (Difco, Detroit, MI). The splenocytes were harvested 5 days and prepared in RPMI medium supplemented with 10% FCS and antibiotics in a flat bottom 96-well plate with 1x10⁶ cells/well. The splenocytes were then stimulated with a combination of IFNγ (60 ng/mL) and SAC (0.025%) [or LPS (20 µg/mL)] in the presence of a test compound. Cell-free supernatants were taken 24 h later for the measurement of cytokines. The preparation of compound and the assessment of cell viability were carried out as described above. Mouse IL-12 p70, IL-10, IL-1 β, and TNFα were measured using ELISA kits from Endogen, according to the manufacturer's instructions.

The biological activities of pyrimidine compounds were tested on human PBMC or THP-1 cells. Unexpectedly, some of the test compounds have IC₅₀ values as low as < 1 nM.

### Example 42. In vivo assays

*Treatment of adjuvant arthritis in rats:* Adjuvant arthritis (AA) was induced in female Lewis rats by the intracutaneous injection (base of the tail) of 0.1 mL of a 10 mg/mL bacterial suspension made from ground, heat-killed *Mycobacterium tuberculosis* H37Ra suspended in incomplete Freund's adjuvant. Rats were given a test compound orally once a day for 12 days, starting the day following the induction. The development of polyarthritis was monitored daily by macroscopic inspection and assignment of an arthritis index to each animal, during the critical period (days 10 to 25 post-immunization).

The intensity of polyarthritis was scored according to the following scheme: (a) Grade each paw from 0 to 3 based on erythema, swelling, and deformity of the joints: 0 for no erythema or swelling; 0.5 if swelling is detectable in at least one joint; 1 for mild swelling and erythema; 2 for swelling and erythema of both tarsus and carpus; and 3 for ankylosis and bony deformity. Maximum score for all 4 paws was thus 12. (b) Grade for other parts of the body: for each ear, 0.5 for redness and another 0.5 if knots are present; 1 for connective tissue swelling (saddle nose); and 1 for the presence of knots or kinks in the tail. The highest possible arthritic index was 16.

Oral administration of pyrimidine compounds of this invention (e.g., Compound 12) reproducibly reduced the arthritic score and delayed the development of polyarthritis in a dose-dependent manner. The arthritis score used in this model was a reflection of the inflammatory state of the structures monitored and the results therefore show the ability of the test compound to provide relief for this aspect of the pathology.

*Treatment of Crohn's disease in dinitrobenzene sulfonic acid-induced inflammatory bowel syndrome model rats:* Wistar derived male or female rats weighing 200± 20 g and fasted for 24 hours were used. Distal colitis was induced by intra-colonic instillation of 2,4-dinitrobenzene sulfonic acid (DNBS, 25 mg in 0.5 mL ethanol 30%) after which air (2 mL) was gently injected through the cannula to ensure that the solution remained in the colon. A test compound and/or vehicle was administered orally 24 and 2 hours before DNBS instillation and then daily for 5 days. One control group was similarly treated with vehicle alone while the other is treated with vehicle plus DNBS. The animals were sacrificed 24 hours after the final dose of test compound administration and each colon was removed and weighed. Colon-to-body weight ratio was then calculated for each animal according to the formula: Colon (g)/BW × 100. The "Net" increase in ratio of Vehicle-control + DNBS group relative to Vehicle-control group was used as a base for comparison with test substance treated groups and expressed as "% Deduction." Pyrimidine compounds of this invention (e.g., Compound 12) reproducibly had about 30% deduction. A 30% or more reduction in colon-to-body weight ratio, relative to the vehicle treated control group, was considered significant.

Rats treated with test substance orally showed a marked reduction in the inflammatory response. These experiments were repeated three times and the effects were reproducible.

*Treatment of Crohn's disease in CD4⁺ CD45Rb^{high} T cell-reconstituted SCID colitis model mice:* Spleen cells were prepared from normal female BALB/c mice. For cell purification, the following anti-mouse antibodies were used to label non-CD4⁺ T cells: B220 (RA3-6B2), CD11b (M1/70), and CD8α (53-6.72). All antibodies were obtained from BioSource (Camarillo, CA). M450 anti-rat IgG-coated magnetic beads (Dynal, Oslo, Norway) were used to bind the antibodies and negative selection was accomplished using an MPC-1 magnetic concentrator. The enriched CD4⁺ cells were then labeled for cell sorting with FITC-conjugated CD45RB (16A, Pharmingen, San Diego, CA) and PE-conjugated CD4 (CT-CD4, Caltag, Burlingame, CA). CD4⁺ CD45RB^{high} cells were operationally defined as the upper 40% of CD45Rb-staining CD4⁺ cells and sorted under sterile conditions by flow cytometry. Harvested cells were resuspended at 4×10⁶/mL in PBS and injected 100 µL intraperitoneally into female C.B-17 *SCID* mice. Pyrimidine compounds of this invention (e.g., Compound 12) and/or vehicle was orally administered once a day, 5 days per week, starting the day following the transfer. The transplanted SCID mice were weighed weekly and their clinical condition was monitored.

Colon tissue samples were fixed in 10% buffered formalin and embedded in paraffin. Sections (4 µm) collected from ascending, transverse, and descending colon were cut and stained with hematoxylin and eosin. The severity of colitis was determined based on histological examination of the distal colon sections, whereby the extent of colonic inflammation was graded on a scale of 0-3 in each of four criteria: crypt elongation, cell infiltration, depletion of goblet cells, and the number of crypt abscesses.

LP lymphocytes were isolated from freshly obtained colonic specimens. After removal of payer's patches, the colon was washed in Ca/Mg-free HBSS, cut into 0.5 cm pieces and incubated twice in HBSS containing EDTA (0.75 mM), DTT (1 mM), and antibiotics (amphotericin 2.5 µg/mL, gentamicin 50 µg/mL from Sigma) at 37°C for 15 min. Next, the tissue was digested further in RPMI containing 0.5 mg/mL collagenase D, 0.01 mg/mL DNase I (Boehringer Manheim), and antibiotics at 37°C. LP cells were then layered on a 40-100% Percoll gradient (Pharmacia, Uppsala, Sweden), and lymphocyte-enriched populations were isolated from the cells at the 40-100% interface.

To measure cytokine production, 48-well plates were coated with 10 µg/mL murine anti-CD3e antibody (145-2C11) in carbonate buffer (PH 9.6 overnight at 4°C. 5× 10⁵ LP cells were then cultured in 0.5ml of complete medium in precoated wells in the presence of 1 µg/mL soluble anti-CD28 antibody (37.51). Purified antibodies were obtained from Pharmingen. Culture supernatants were removed after 48 h and assayed for cytokine production. Murine IFNγ was measured using an ELISA kit from Endogen (Cambridge, MA), according to the manufacturer's instructions.

Histological analysis showed that oral administration of pyrimidine compounds of this invention (e.g., Compound 12) reduced colonic inflammation as compared to vehicle control. The suppressive effect was dose-dependent with a substantial reduction at a dose of 10 mg/kg. The calculated colon-to-body weight ratio was consistent with the histological score, showing attenuation by treatment with the test compound. Furthermore, analysis of cytokines from LP cells in response to anti-CD3 antibody and anti-CD28 antibody demonstrated that LP cells from vehicle control produced an augmented level of IFNγ and treatment with test substance greatly diminished the production. These results clearly demonstrated the potential of the test substance in treatment of inflammatory bowel disease represented by Crohn's disease.

### OTHER EMOBDIMENTS

All of the features disclosed in this specification may be combined in any combination. Each feature disclosed in this specification may be replaced by an alternative feature serving the same, equivalent, or similar purpose. Thus, unless expressly stated otherwise, each feature disclosed is only an example of a generic series of equivalent or similar features.

## Claims

1. A compound of formula (I) 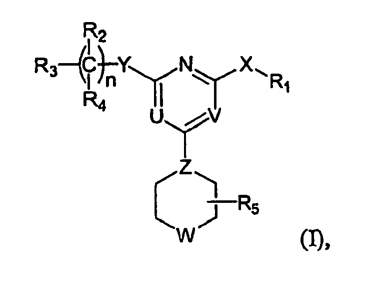 wherein 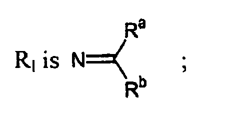 each of R₂ and R₄, independently, is R^{c}, halogen, nitro, cyano, isothionitro, SR^{c}, or OR^{c}; or R₂ and R₄, taken together, is carbonyl;
R₃ is R^{c}, alkenyl, alkynyl, OR^{c}, OC(O)R^{c}, SO₂R^{c}, S(O)R^{c}, S(O₂)NR^{c}R^{d}, SR^{c}, NR^{c}R^{d}, NR^{c}COR^{d}, NR^{c}C(O)OR^{d}, NR^{c}C(O)NR^{c}R^{d}, NR^{c}SO₂R^{d}, COR^{c}, C(O)OR^{c}, or C(O)NR^{c}R^{d};
R₅ is H or alkyl;
n is 0, 1, 2, 3, 4, 5, or 6;
X is O, S, S(O), S(O₂), or NR^{c};
Y is a covalent bond, CH₂, C(O), C=N-R^{c}, C=N-OR^{c}, C=N-SR^{c}, O, S, S(O), S(O₂), or NR^{c};
Z is N or CH;
one of U and V is N, and the other is CR^{c}; and
W is O, S, S(O), S(O₂), NR^{c}, or NC(O)R^{c};
in which each of R^{a} and R^{b}, independently, is H, alkyl, aryl, heteroaryl; and each of R^{c} and R^{d}, independently, is H, alkyl, aryl, heteroaryl, cyclyl, heterocyclyl, or alkylcarbonyl,
wherein alkyl refers to an alkyl group containing up to 6 carbon atoms, alkenyl refers to an alkenyl group containing up to 6 carbon atoms, and alkynyl refers to an alkynyl group containing up to 6 carbon atoms, and
wherein the alkyl, alkenyl, alkynyl, aryl, heteroaryl, cyclyl, and heterocyclyl groups are optionally substituted.

2. The compound of claim 1, wherein U is N and V is CH.

3. The compound of claim 1, wherein Z is N and W is O.

4. The compound of claim 1, wherein X is O or NR^{c}.

5. The compound of claim 1, wherein Y is a covalent bond, O, S, or CH₂, and n is 0, 1, 2, 3, or 4.

6. The compound of claim 5, wherein R₃ is aryl, heteroaryl, OR^{c}, SR^{c}, C(O)OR^{c}, NR^{c}R^{d}, or C(O)NR^{c}R^{d}.

7. The compound of claim 5, wherein R₃ is 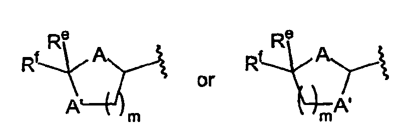 in which
each of A and A', independently, is O, S, or NH;
each of R^{e} and R^{f} independently, is H, alkyl, aryl, or heteroaryl; and
m is 1 or 2,
wherein alkyl refers to an alkyl group containing up to 6 carbon atoms, and
wherein the alkyl, aryl, and heteroaryl groups are optionally substituted.

8. The compound of claim 1, wherein one of R^{a} and R^{b} is 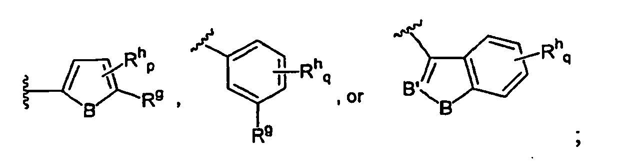 in which
B is NRⁱ, O, or S;
B' is N or CRⁱ;
R^{g} is H, halogen, CN, alkyl, cyclyl, alkyloxy, alkylcarbonyl, alkyloxycarbonyl, aryloxycarbonyl, heteroaryloxycarbonyl, hydroxyalkyl, alkylamino, or alkylaminocarbonyl;
R^{h} is H, halogen, NO₂, CN, alkyl, aryl, heteroaryl, OR^{c}, OC(O)R^{c}, SO₂R^{c}, S(O)R^{c}, S(O₂)NR^{c}R^{d}, SR^{c}, NR^{c}R^{d}, NR^{c}COR^{d}, NR^{c}C(O)OR^{d}, NR^{c}C(O)NR^{c}R^{d}, NR^{c}SO₂R^{d}, COR^{c}, C(O)OR^{c}, or C(O)NR^{c}R^{d}
R¹ is H, alkyl, or alkylcarbonyl;
p is 0, 1, or 2; and
q is 0, 1, 2, 3, or 4,
wherein alkyl refers to an alkyl group containing up to 6 carbon atoms, and
wherein the alkyl, aryl, heteroaryl, and cyclyl groups are optionally substituted.

9. The compound of claim 8, wherein one of R^{a} and R^{b} is 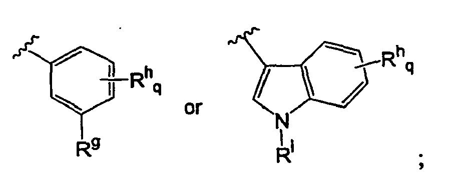 in which
R^{g}, R^{h}, Rⁱ, and q are as defined in claim 8; and
the other of R^{a} and R^{b} is H or alkyl.

10. The compound of claim 9, wherein
R^{g} is H, methyl, ethyl, propyl, cyclopropyl, methoxy, ethoxy, methoxycarbonyl, or halogen;
R^{h} is F, Cl, CN, methyl, methoxy, ethoxy, OC(O)CH₃, OC(O)C₂H₅, C(O)OH, C(O)OC₂H₅, C(O)NH₂, NHC(O)CH₃, or S(O₂)NH₂;
R¹ is H, methyl, ethyl, or acetyl; and
q is 0, 1, or 2.

11. The compound of claim 10, wherein U is N, V is CH, Z is N, and W is O.

12. The compound of claim 11, wherein X is NR^{c}; and R^{c} is H, methyl, ethyl, or acetyl.

13. The compound of claim 12, wherein Y is a covalent bond, O, S, or CH₂; and n is 0, 1, 2, 3, or 4.

14. The compound of claim 13, wherein R₃ is R^{c}, OR^{c}, SR^{c}, C(O)OR^{c}, or C(O)NR^{c}R^{d}.

15. The compound of claim 14, wherein R₃ aryl, heteroaryl, hydroxyl, alkyloxy, or heteroaryloxy.

16. The compound of claim 1, wherein
each of R₂ and R₄ is H;
R₃ is H, alkyl, aryl, heteroaryl, cyclyl, heterocyclyl, alkyloxycarbonyl, alkylaminocarbonyl, or alkylcarbonyl; and
X is NR^{c}_{.}

17. The compound of claim 16, wherein X is NH.

18. The compound of claim 16, wherein one of R^{a} and R^{b} is H or alkyl; and the other is aryl or heteroaryl optionally substituted with R^{g}_{q} and R^{h}; R^{g} being halogen, CN, alkyl, alkyloxy, alkylcarbonyl, alkyloxycarbonyl, aryloxycarbonyl, heteroaryloxycarbonyl, hydroxyalkyl, alkylamino, or alkylaminocarbonyl; R^{h} being halogen, CN, hydroxyl, alkyl, aryl, heteroaryl, alkoxyl, aryloxyl, or heteroaryloxyl; and q being 0, 1, 2, 3, or 4.

19. The compound of claim 18, wherein one of R^{a} and R^{b} is H or alkyl; and the other is 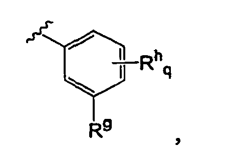 in which
R^{g} is H, alkyl, alkoxyl, methoxycarbonyl, or halogen;
R^{h} is halogen, CN, hydroxyl, alkyl, aryl, heteroaryl, alkoxyl, aryloxyl, or heteroaryloxyl; and
q is 0, 1, 2, 3, or 4,
wherein alkyl refers to an alkyl group containing up to 6 carbon atoms, and
wherein the alkyl, aryl, and heteroaryl groups are optionally substituted.

20. The compound of claim 16, wherein U is N, V is CH, Z is N, and W is O.

21. The compound of claim 20, wherein R₃ is heteroaryl or heterocyclyl.

22. The compound of claim 21, wherein R₃ is pyridinyl, 1-oxy-pyridinyl, or 1*H*-pyridin-2-one.

23. The compound of claim 21, wherein n is 2, and Y is O.

24. The compound of claim 23, wherein X is NH.

25. The compound of claim 24, wherein one of R^{a} and R^{b} is H or alkyl; and the other is 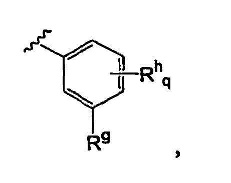 in which
R^{g} is H, alkyl, alkoxyl, methoxycarbonyl, or halogen;
R^{h} is halogen, CN, hydroxyl, alkyl, aryl, heteroaryl, alkoxyl, aryloxyl, or heteroaryloxyl; and
q is 0, 1, 2, 3, or 4,
wherein alkyl refers to an alkyl group containing up to 6 carbon atoms, and
wherein the alkyl, aryl, and heteroaryl groups are optionally substituted.

26. The compound of claim 25, wherein one of R^{a} and R^{b} is H; and the other is 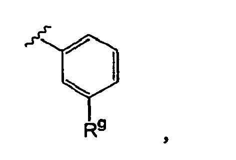 in which R^{g} is as defined in claim 25.

27. The compound of claim 1, wherein the compound is:
N-{2-[3-(3,4-dimethoxy-phenyl)-propyl]-6-morpholin-4-yl-pyrimidin-4-yl}-N'-(1-H-indol-3-ylmethylene)-hydrazine,
N-(2-n-butoxy-6-morpholin-4-yl-pyrimidin-4-yl)-N'-(1H-indol-3-ylmethylene)-hydrazine,
N-(2-(4-hydroxybutyl)-6-morpholin-4-yl-pyrimidin-4-yl)-N'-(1H-indol-3-ylmethylene)-hydrazine,
N-[2-(2-[1,3]dioxan-2-yl-ethyl)-6-morpholin-4-yl-pyrimidin-4-yl]-N'-(1H-indol-3-ylmethylene)-hydrazine,
N-(1H-indol-3-ylmethylene)-N'-[2-(3-methoxy-propyl)-6-morpholin-4-yl-pyrimidin-4-yl]-hydrazine,
3-{4-[N'-(1H-indol-3-ylmethylene)-hydrazino]-6-morpholin-4-yl-pyrimidin-2-ylsulfanyl}-propan-1-ol,
3-{2-[N'-(1H-indol-3-ylmethylene)-hydrazino]-6-morpholin-4-yl-pyrimidin-4-ylsulfanyl}-propan-1-ol,
N-[2-(2,2-dimethyl-[1,3]dioxolan-4-ylmethoxy)-6-morpholin-4-yl-pyrimidin-4-yl]-N'-(1H-indol-3-ylmethylene)-hydrazine,
N- {2-[2-(3,4-dimethoxy-phenyl)-ethoxy]-6-morpholin-4-yl-pyrimidin-4-yl}-N'-(1H-indol-3-ylmethylene)-hydrazine,
N-(1H-indol-3-ylmethylene)-N'-[6-morpholin-4-yl-2-(2-pyridin-2-yl-ethoxy)-pyrimidin-4-yl]-hydrazine,
N-(1H-indol-3-ylmethylene)-N'-[6-morpholin-4-yl-2-(3-pyridin-2-yl-propyl)-pyrimidin-4-yl]-hydrazine,
N-(3-methyl-benzylidene)-N'-[6-morpholin-4-yl-2-(2-pyridin-2-yl-ethoxy)-pyrimidin-4-yl]-hydrazine,
N-(3-ethyl-benzylidene)-N'-[6-morpholin-4-yl-2-(2-pyridin-2-yl-ethoxy)-pyrimidin-4-yl]-hydrazine,
N-(3-methyl-benzylidene)-N'-[6-morpholin-4-yl-2-(3-pyridin-2-yl-propyl)-pyrimidin-4-yl]-hydrazine,
N-[6-morpholin-4-yl-2-(2-pyridin-2-yl-ethoxy)-pyrimidin-4-yl]-N'-(1-*m*-tolyl-ethylidene)-hydrazine,
N-[1-(1H-indol-3-yl)-ethylidene]-N'-[6-morpholin-4-yl-2-(2-pyridin-2-yl-ethoxy)-pyrimidin-4-yl]-hydrazine,
3-methyl-benzaldehyde O-[6-morpholin-4-yl-2-(2-pyridin-2-yl-ethoxy)-pyrimidin-4-yl]-oxime,
1H-indole-3-carbaldehyde O-[6-morpholin-4-yl-2-(2-pyridin-2-yl-ethoxy)-pyrim-idin-4-yl]-oxime,
N-(1H-indol-3-ylmethylene)-N'-{6-morpholin-4-yl-2-[2-(pyridin-3-yloxy)-ethoxy]-pyrimidin-4-yl}-hydrazine,
N-(3-methyl-benzylidene)-N'-{6-morpholin-4-yl-2-[2-(pyridin-3-yloxy)-ethoxy]-pyrimidin-4-yl}-hydrazine,
butyl-{4-[N'-(1H-indol-3-ylmethylene)-hydrazino]-6-morpholin-4-yl-pyrimidin-2-yl}-amine,
N-(3-methyl-benzylidene)-N'-[6-morpholin-4-yl-2-(pyridin-3-yloxy)-pyrimidin-4-yl]-hydrazine,
N-(3-methylbenzylidene)-N'-(5-methyl-6-morpholin-4-yl-2-phenylpyrimidin-4-yl)hydrazine,
N-(3-methyl-benzylidene)-N'-(2-phenyl-6-thiomorpholin-4-yl-pyrimidin-4-yl)-hydrazine,
3-{4-[N'-(3-methyl-benzylidene)-hydrazino]-6-morpholin-4-yl-pyrimidin-2-yl}-propionic acid ethyl ester,
N-(3-methyl-benzylidene)-N'- {6-morpholin-4-yl-2-[2-(-oxy-pyridin-2-yl)-ethoxy]-pyrimidin-4-yl}-hydrazine,
1-(2-{4-[N'-(3-methyl-benzylidene)-hydrazino]-6-morpholin-4-yl-pyrimidin-2-yloxy}-ethyl)-1H-pyridin-2-one,
N-(3-iodo-benzylidene)-N'-[6-morpholin-4-yl-2-(2-pyridin-2-yl-ethoxy)-pyrimidin-4-yl]-hydrazine,
N-(3-fluoro-benzylidene)-N'-[6-morpholin-4-yl-2-(2-pyridin-2-yl-ethoxy)-pyrimidin-4-yl]-hydrazine,
N-(3-chloro-benzylidene)-N'-[6-morpholin-4-yl-2-(2-pyridin-2-yl-ethoxy)-pyrimidin-4-yl]-hydrazine,
N-(3-bromo-benzylidene)-N'-[6-morpholin-4-yl-2-(2-pyridin-2-yl-ethoxy)-pyrimidin-4-yl]-hydrazine,
3-{[6-morpholin-4-yl-2-(2-pyridin-2-yl-ethoxy)-pyrimidin-4-yl]-hydrazonomethyl}-benzoic acid methyl ester,
1-(2-{4-[N'-(3-iodo-benzylidene)-hydrazino]-6-morpholin-4-yl-pyrimidin-2-yloxy}-ethyl)-1H-pyridin-2-one,
3-{[6-morpholin-4-yl-2-(2-pyridin-2-yl-ethoxy)-pyrimidin-4-yl]-hydrazonomethyl}-benzoic acid N-methyl amide,
(3-{[6-morpholin-4-yl-2-(2-pyridin-2-yl-ethoxy)-pyrimidin-4-yl]-hydrazonomethyl}-phenyl)-methanol,
N,N-diethyl-4-{4-[N"-(3-methyl-benzylidene)-hydrazino]-6-morpholin-4-yl-pyrimidin-2-yl -butyramide,
4-{4-[N'-(3-methyl-benzylidene)-hydrazino]-6-morpholin-4-yl-pyrimidin-2-yl}-1-(4-methyl-piperazin-1-yl)-butan-1-one,
4-{4-[N'-(3-methyl-benzylidene)-hydrazino]-6-morpholin-4-yl-pyrimidin-2-yl}-N-pyridin-4-ylmethyl-butyramide, or
4-{4-[N'-(3-methyl-benzylidene)-hydrazino]-6-morpholin-4-yl-pyrimidin-2-yl}-N-pyridin-4-yl-butyramide.

28. A pharmaceutical composition comprising a pharmaceutically acceptable carrier and an effective amount of a compound of any one of claims 1 to 27.

29. Use of a compound of any one of claims 1 to 27 for the preparation of a pharmaceutical composition for treating an interleukin-12 overproduction-related disorder.

30. The use of claim 29, wherein the disorder is rheumatoid arthritis, sepsis, Crohn's disease, multiple sclerosis, psoriasis, or insulin-dependent diabetes mellitus.

## Patentansprüche

1. Verbindung der Formel (I) 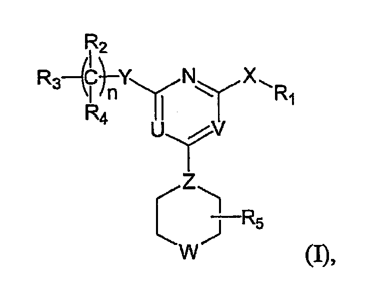 worin
R₁ 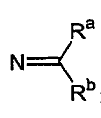 ist;
jeder der Substituenten R₂ und R₄ unabhängig voneinander eine R^{c}-Gruppe, ein Halogenatom, eine Nitro-, Cyan-, Isothionitro-, SR^{c}- oder OR^{c}-Gruppe ist, oder R₂ und R₄ zusammengenommen eine Carbonylgruppe sind;
R₃ eine R^{c}-, Alkenyl-, Alkinyl-, OR^{c}-, OC(O)R^{c}-, SO₂R^{c}-, S(O)R^{c}-, S(O₂)NR^{c}R^{d}-, SR^{c}-, NR^{c}R^{d}-, NR^{c}COR^{d}-, NR^{c}C(O)OR^{d}-, NR^{c}C(O)NR^{c}R^{d}-, NR^{c}SO₂R^{d}-, COR^{c}-, C(O)OR^{c}- oder C(O)NR^{c}R^{d}-Gruppe ist;
R₅ ein H-Atom oder eine Alkylgruppe ist;
n den Wert 0, 1, 2, 3, 4, 5 oder 6 aufweist;
X ein O-, S-Atom, eine S(O)-, S(O₂)- oder NR^{c}-Gruppe ist;
Y eine kovalente Bindung, eine CH₂-, C(O)-, C=N-R^{c}-, C=N-OR^{c}-, C=N-SR^{c}-Gruppe, ein O-, S-Atom, eine S(O)-, S(O₂) oder NR^{c}-Gruppe ist;
Z ein N-Atom oder eine CH-Gruppe ist;
einer der Substituenten U und V ein N-Atom ist und der andere eine CR^{c}-Gruppe ist; und
W ein O-, S-Atom, eine S(O)-, S(O₂)-, NR^{c}- oder NC(O)R^{c}-Gruppe ist; wobei jeder der Substituenten R^{a} und R^{b} unabhängig voneinander ein H-Atom, eine Alkyl-, Aryl-, Heteroarylgruppe ist, und jeder der Substituenten R^{c} und R^{d} unabhängig voneinander ein H-Atom, eine Alkyl-, Aryl-, Heteroaryl-, Cyclyl-, Heterocyclyl- oder Alkylcarbonylgruppe ist,
wobei eine Alkylgruppe eine Alkylgruppe mit bis zu 6 Kohlenstoffatomen betrifft, eine Alkenylgruppe eine Alkenylgruppe mit bis zu 6 Kohlenstoffatomen betrifft und eine Alkinylgruppe eine Alkinylgruppe mit bis zu 6 Kohlenstoffatomen betrifft, und
wobei die Alkyl-, Alkenyl-, Alkinyl-, Aryl-, Heteroaryl-, Cyclyl- und Heterocyclylgruppen gegebenenfalls substituiert sind.

2. Verbindung nach Anspruch 1, wobei U ein N-Atom ist und V eine CH-Gruppe ist.

3. Verbindung nach Anspruch 1, wobei Z ein N-Atom ist und W ein O-Atom ist.

4. Verbindung nach Anspruch 1, wobei X ein O-Atom oder eine NR^{c}-Gruppe ist.

5. Verbindung nach Anspruch 1, wobei Y eine kovalente Bindung, ein O-, S-Atom oder eine CH₂-Gruppe ist, und n den Wert 0, 1, 2, 3 oder 4 aufweist.

6. Verbindung nach Anspruch 5, wobei R₃ eine Aryl-, Heteroaryl-, OR^{c}-, SR^{c}-, C(O)OR^{c}-, NR^{c}R^{d}- oder C(O)NR^{c}R^{d}-Gruppe ist.

7. Verbindung nach Anspruch 5, wobei R₃ 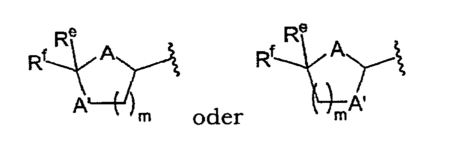 ist,
worin
jeder der Substituenten A und A' unabhängig voneinander ein O-, S-Atom oder eine NH-Gruppe ist;
jeder der Substituenten R^{e} und R^{f} unabhängig voneinander ein H-Atom, eine Alkyl-, Aryl- oder Heteroarylgruppe ist; und
m den Wert 1 oder 2 aufweist,
wobei eine Alkylgruppe eine Alkylgruppe mit bis zu 6 Kohlenstoffatomen betrifft, und
wobei die Alkyl-, Aryl- und Heteroarylgruppen gegebenenfalls substituiert sind.

8. Verbindung nach Anspruch 1, wobei einer der Substituenten R^{a} und R^{b} 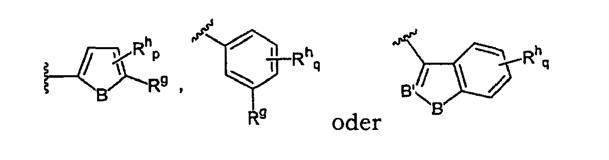 ist,
worin
B eine NRⁱ-Gruppe, ein O- oder S-Atom ist;
B' ein N-Atom oder eine CRⁱ-Gruppe ist;
R^{g} ein H-, Halogenatom, eine CN-, Alkyl-, Cyclyl-, Alkyloxy-, Alkylcarbonyl-, Alkyloxycarbonyl-, Aryloxycarbonyl-, Heteroaryloxycarbonyl-, Hydroxyalkyl-, Alkylamino- oder Alkylaminocarbonylgruppe ist;
R^{h} ein H-, Halogenatom, eine NO₂-, CN-, Alkyl-, Aryl-, Heteroaryl-, OR^{c}-, OC(O)R^{c}-, SO₂R^{c}-, S(O)R^{c}-, S(O₂)NR^{c}R^{d}-, SR^{c}-, NR^{c}R^{d}-, NR^{c}COR^{d}-, NR^{c}C(O)OR^{d}-, NR^{c}C(O)NR^{c}R^{d}-, NR^{c}SO₂R^{d}-, COR^{c}-, C(O)OR^{c}- oder C(O)NR^{c}R^{d}-Gruppe ist;
Rⁱ ein H-Atom, eine Alkyl- oder Alkylcarbonylgruppe ist;
p den Wert 0, 1 oder 2 aufweist; und
q den Wert 0, 1, 2, 3 oder 4 aufweist,
wobei eine Alkylgruppe eine Alkylgruppe mit bis zu 6 Kohlenstoffatomen betrifft, und
wobei die Alkyl-, Aryl-, Heteroaryl- und Cyclylgruppen gegebenenfalls substituiert sind.

9. Verbindung nach Anspruch 8, wobei einer der Substituenten R^{a} und R^{b} 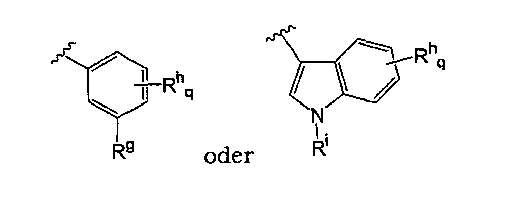 ist,
worin
R^{g}, R^{h}, Rⁱ und q wie in Anspruch 8 definiert sind; und
der andere der Substituenten R^{a} und R^{b} ein H-Atom oder eine Alkylgruppe ist.

10. Verbindung nach Anspruch 9, wobei
R^{g} ein H-Atom, eine Methyl-, Ethyl-, Propyl-, Cyclopropyl-, Methoxy-, Ethoxy-, Methoxycarbonylgruppe oder ein Halogenatom ist;
R^{h} ein F-, Cl-Atom, eine CN-, Methyl-, Methoxy-, Ethoxy-, OC(O)CH₃-, OC(O)C₂H₅-, C(O)OH-, C(O)OC₂H₅-, C(O)NH₂-, NHC(O)CH₃- oder S(O₂)NH₂ Gruppe ist;
Rⁱ ein H-Atom, eine Methyl-, Ethyl- oder Acetylgruppe ist; und
q den Wert 0, 1 oder 2 aufweist.

11. Verbindung nach Anspruch 10, wobei U ein N-Atom ist, V eine CH-Gruppe ist, Z ein N-Atom ist und W ein O-Atom ist.

12. Verbindung nach Anspruch 11, wobei X eine NR^{c}-Gruppe ist und R^{c} ein H-Atom, eine Methyl-, Ethyl- oder Acetylgruppe ist.

13. Verbindung nach Anspruch 12, wobei Y eine kovalente Bindung, ein O-, S-Atom oder eine CH₂-Gruppe ist, und n den Wert 0, 1, 2, 3 oder 4 aufweist.

14. Verbindung nach Anspruch 13, wobei R₃ eine R^{c}-, OR^{c}-, SR^{c}-, C(O)OR^{c}-oder C(O)NR^{c}R^{d}-Gruppe ist.

15. Verbindung nach Anspruch 14, wobei R₃ eine Aryl-, Heteroaryl-, Hydroxyl-, Alkyloxy- oder Heteroaryloxygruppe ist.

16. Verbindung nach Anspruch 1, wobei
jeder der Substituenten R₂ und R₄ ein H-Atom ist;
R₃ ein H-Atom, eine Alkyl-, Aryl-, Heteroaryl-, Cyclyl-, Heterocyclyl-, Alkyloxycarbonyl-, Alkylaminocarbonyl- oder Alkylcarbonylgruppe ist; und
X eine NR^{c}-Gruppe ist.

17. Verbindung nach Anspruch 16, wobei X eine NH-Gruppe ist.

18. Verbindung nach Anspruch 16, wobei einer der Substituenten R^{a} und R^{b} ein H-Atom oder eine Alkylgruppe ist und der andere eine Aryl- oder Heteroarylgruppe, die gegebenenfalls mit R^{g}_{q} und R^{h} substituiert ist, ist, wobei R^{g} ein Halogenatom, eine CN-, Alkyl-, Alkyloxy-, Alkylcarbonyl-, Alkyloxycarbonyl-, Aryloxycarbonyl-, Heteroaryloxycarbonyl-, Hydroxyalkyl-, Alkylamino- oder Alkylaminocarbonylgruppe ist; R^{h} ein Halogenatom, eine CN-, Hydroxyl-, Alkyl-, Aryl-, Heteroaryl-, Alkoxyl-, Aryloxyl- oder Heteroaryloxylgruppe ist; und q den Wert 0, 1, 2, 3 oder 4 aufweist.

19. Verbindung nach Anspruch 18, wobei einer der Substituenten R^{a} und R^{b}
ein H-Atom oder eine Alkylgruppe ist und der andere 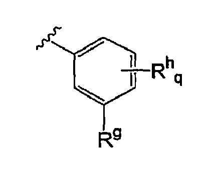 ist,
worin
R^{g} ein H-Atom, eine Alkyl-, Alkoxyl-, Methoxycarbonylgruppe oder ein Halogenatom ist;
R^{h} ein Halogenatom, eine CN-, Hydroxyl-, Alkyl-, Aryl-, Heteroaryl-, Alkoxyl-, Aryloxyl- oder Heteroaryloxylgruppe ist; und
q den Wert 0, 1, 2, 3 oder 4 aufweist,
wobei eine Alkylgruppe eine Alkylgruppe mit bis zu 6 Kohlenstoffatomen betrifft, und
wobei die Alkyl-, Aryl- und Heteroarylgruppen gegebenenfalls substituiert sind.

20. Verbindung nach Anspruch 16, wobei U ein N-Atom ist, V eine CH-Gruppe ist, Z ein N-Atom ist und W ein O-Atom ist.

21. Verbindung nach Anspruch 20, wobei R₃ eine Heteroaryl- oder Heterocyclylgruppe ist.

22. Verbindung nach Anspruch 21, wobei R₃ eine Pyridinyl-, 1-Oxy-pyridinyl- oder 1*H*-Pyridin-2-on-Gruppe ist.

23. Verbindung nach Anspruch 21, wobei n den Wert 2 aufweist und Y ein O-Atom ist.

24. Verbindung nach Anspruch 23, wobei X eine NH-Gruppe ist.

25. Verbindung nach Anspruch 24, wobei einer der Substituenten R^{a} und R^{b}
ein H-Atom oder eine Alkylgruppe ist und der andere 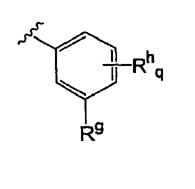 ist,
worin
R^{g} ein H-Atom, eine Alkyl-, Alkoxyl-, Methoxycarbonylgruppe oder ein Halogenatom ist;
R^{h} ein Halogenatom, eine CN-, Hydroxyl-, Alkyl-, Aryl-, Heteroaryl-, Alkoxyl-, Aryloxyl- oder Heteroaryloxylgruppe ist; und
q den Wert 0, 1, 2, 3 oder 4 aufweist,
wobei eine Alkylgruppe eine Alkylgruppe mit bis zu 6 Kohlenstoffatomen betrifft, und
wobei die Alkyl-, Aryl- und Heteroarylgruppen gegebenenfalls substituiert sind.

26. Verbindung nach Anspruch 25, wobei einer der Substituenten R^{a} und R^{b}
ein H-Atom ist und der andere 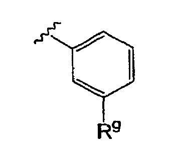 ist,
worin R^{g} wie in Anspruch 25 definiert ist.

27. Verbindung nach Anspruch 1, wobei die Verbindung
N-{2-[3-(3,4-Dimethoxy-phenyl)-propyl]-6-morpholin-4-yl-pyrimidin-4-yl}-N'-(1H-indol-3-ylmethylen)-hydrazin,
N-(2-n-Butoxy-6-morpholin-4-yl-pyrimidin-4-yl)-N'-(1H-indol-3-yl-methylen)-hydrazin,
N-(2-(4-Hydroxybutyl)-6-morpholin-4-yl-pyrimidin-4-yl)-N'-(1H-indol-3-ylmethylen)-hydrazin,
N-[2-(2-[1,3]Dioxan-2-yl-ethyl)-6-morpholin-4-yl-pyrimidin-4-yl]-N'-(1H-indol-3-ylmethylen)-hydrazin,
N-(1H-Indol-3-ylmethylen)-N'-[2-(3-methoxy-propyl)-6-morpholin-4-yl-pyrimidin-4-yl]-hydrazin,
3-{4-[N'-(1H-Indol-3-ylmethylen)-hydrazino]-6-morpholin-4-yl-pyrimidin-2-ylsulfanyl}-propan-1-ol,
3-{2-[N'-(1H-Indol-3-ylmethylen)-hydrazino]-6-morpholin-4-yl-pyrimidin-4-ylsulfanyl}-propan-1-ol,
N-[2-(2,2-Dimethyl-[1,3]dioxolan-4-ylmethoxy)-6-morpholin-4-yl-pyrimidin-4-yl]-N'-(1H-indol-3-ylmethylen)-hydrazin
N-{2-[2-(3,4-Dimethoxy-phenyl)-ethoxy]-6-morpholin-4-yl-pyrimidin-4-yl}-N'-(1H-indol-3-ylmethylen)-hydrazin,
N-(1H-Indol-3-ylmethylen)-N'-[6-morpholin-4-yl-2-(2-pyridin-2-yl-ethoxy)-pyrimidin-4-yl]-hydrazin,
N-(1H-Indol-3-ylmethylen)-N'[6-morpholin-4-yl-2-(3-pyridin-2-yl-propyl)-pyrimidin-4-yl]-hydrazin,
N-(3-Methyl-benzyliden)-N'-[6-morpholin-4-yl-2-(2-pyridin-2-yl-ethoxy)-pyrimidin-4-yl]-hydrazin,
N-(3-Ethyl-benzyliden)-N'-[6-morpholin-4-yl-2-(2-pyridin-2-yl-ethoxy)-pyrimidin-4-yl]-hydrazin,
N-(3-Methyl-benzyliden)-N'-[6-morpholin-4-yl-2-(3-pyridin-2-yl-propyl)-pyrimidin-4-yl]-hydrazin,
N-[6-Morpholin-4-yl-2-(2-pyridin-2-yl-ethoxy)-pyrimidin-4-yl]-N'-(1-m-tolyl-ethyliden)-hydrazin,
N-[1-(1H-Indol-3-yl)-ethyliden]-N'-[6-morpholin-4-yl-2-(2-pyridin-2-yl-ethoxy) -pyrimidin-4-yl] -hydrazin,
3-Methyl-benzaldehyd-O-[6-morpholin-4-yl-2-(2-pyridin-2-yl-ethoxy)-pyrimidin-4-yl]-oxim,
1H-Indol-3-carbaldehyd-O-[6-morpholin-4-yl-2-(2-pyridin-2-yl-ethoxy)-pyrimidin-4-yl]-oxim,
N-(1H-Indol-3-ylmethylen)-N'-{6-morpholin-4-yl-2-[2-(pyridin-3-yloxy)-ethoxy]-pyrimidin-4-yl}-hydrazin,
N-(3-Methyl-benzyliden)-N'-{6-morpholin-4-yl-2-[2-(pyridin-3-yloxy)-ethoxy]-pyrimidin-4-yl}-hydrazin,
Butyl-{4-[N'-(1H-indol-3-ylmethylen)-hydrazino]-6-morpholin-4-yl-pyrimidin-2-yl}-amin,
N-(3-Methyl-benzyliden)-N'-[6-morpholin-4-yl-2-(pyridin-3-yloxy)-pyrimidin-4-yl]-hydrazin,
N-(3-Methylbenzyliden)-N'-(5-methyl-6-morpholin-4-yl-2-phenylpyrimidin-4-yl)hydrazin,
N-(3-Methyl-benzyliden)-N'-(2-phenyl-6-thiomorpholin-4-yl-pyrimidin-4-yl)-hydrazin,
3-{4-[N'-(3-Methyl-benzyliden)-hydrazino]-6-morpholin-4-yl-pyrimidin-2-yl}-propionsäureethylester,
N-(3-Methyl-benzyliden)-N'-{6-morpholin-4-yl-2-[2-(1-oxy-pyridin-2-yl)-ethoxy]-pyrimidin-4-yl}-hydrazin,
1-(2-{4-[N'-(3-Methyl-benzyliden)-hydrazino]-6-morpholin-4-yl-pyrimidin-2-yloxy}-ethyl)-1H-pyridin-2-on,
N-(3-Iod-benzyliden)-N'-[6-morpholin-4-yl-2-(2-pyridin-2-yl-ethoxy)-pyrimidin-4-yl]-hydrazin,
N-(3-Fluor-benzyliden)-N'-[6-morpholin-4-yl-2-(2-pyridin-2-yl-ethoxy)-pyrimidin-4-yl]-hydrazin,
N-(3-Chlor-benzyliden)-N'-[6-morpholin-4-yl-2-(2-pyridin-2-yl-ethoxy)-pyrimidin-4-yl]-hydrazin,
N-(3-Brom-benzyliden)-N'-[6-morpholin-4-yl-2-(2-pyridin-2-yl-ethoxy)-pyrimidin-4-yl]-hydrazin,
3-{[6-Morpholin-4-yl-2-(2-pyridin-2-yl-ethoxy)-pyrimidin-4-yl]-hydrazono-methyl}-benzoesäuremethylester,
1-(2-{4-[N'-(3-Iod-benzyliden)-hydrazino]-6-morpholin-4-yl-pyrimidin-2-yloxy}-ethyl)-1H-pyridin-2-on,
3-{[6-Morpholin-4-yl-2-(2-pyridin-2-yl-ethoxy)-pyrimidin-4-yl]-hydrazono-methyl}-benzoesäure-N-methylamid,
(3-{[6-Morpholin-4-yl-2-(2-pyridin-2-yl-ethoxy)-pyrimidin-4-yl]-hydrazono-methyl}-phenyl)-methanol,
N,N-Diethyl-4-{4-[N"-(3-methyl-benzyliden)-hydrazino]-6-morpholin-4-yl-pyrimidin-2-yl}-butyramid,
4-{4-[N'-(3-Methyl-benzyliden)-hydrazino]-6-morpholin-4-yl-pyrimidin-2-yl}-1-(4-methyl-piperazin-1-yl)-butan-1-on,
4-{4-[N'-(3-Methyl-benzyliden)-hydrazino]-6-morpholin-4-yl-pyrimidin-2-yl}-N-pyridin-4-ylmethyl-butyramid oder
4-{4-[N'-(3-Methyl-benzyliden)-hydrazino]-6-morpholin-4-yl-pyrimidin-2-yl}-N-pyridin-4-yl-butyramid ist.

28. Pharmazeutische Zusammensetzung, die einen pharmazeutisch verträglichen Träger und eine wirksame Menge einer Verbindung nach einem jeglichen der Ansprüche 1 bis 27 umfasst.

29. Verwendung einer Verbindung nach einem jeglichen der Ansprüche 1 bis 27 zur Herstellung einer pharmazeutischen Zusammensetzung zum Behandeln einer Erkrankung, die mit einer Interleukin-12-Überproduktion in Beziehung steht.

30. Verwendung nach Anspruch 29, wobei die Erkrankung rheumatoide Arthritis, Sepsis, Morbus Crohn, multiple Sklerose, Psoriasis oder insulinabhängiger Diabetes mellitus ist.

## Revendications

1. Composé de formule (I) 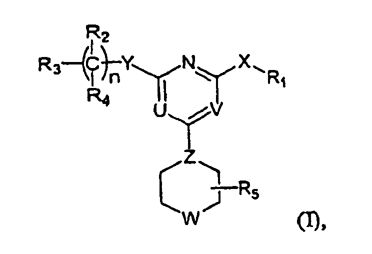 où R₁ est 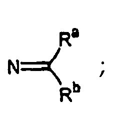 chacun des R₂ et R₄, indépendamment, est R^{c}, halogéno, nitro, cyano, isothionitro, SR^{c}, ou OR^{c}; ou R₂ et R₄, pris ensemble, est le carbonyle;
R₃ est R^{c}, alcényle, alcynyle OR^{c}, OC(O)R^{c}, SO₂R^{c}, S(O)R^{c}, S(O₂)NR^{c}R^{d}, SR^{c}, NR^{c}R^{d}, NR^{c}COR^{d}, NR^{c}C(O)OR^{d}, NR^{c}C(O)NR^{c}R^{d}, NR^{c}SO₂R^{d}, COR^{c}, C(O)OR^{c}, ou C(O)NR^{c}R^{d};
R₅ est H ou alkyle;
n est 0, 1, 2, 3, 4, 5, ou 6;
X est O, S, S(O), S(O₂), ou NR^{c};
Y est une liaison covalente, CH₂, C(O), C=N-R^{c}, C=N-OR^{c}, C=N-SR^{c}, O, S, S(O), S(O₂), ou NR^{c};
Z est N ou CH;
l'un de U et V est N, et l'autre est CR^{c}; et
W est O, S, S(O), S(O₂), NR^{c}, ou NC(O)R^{c};
dans lequel chacun des R^{a} et R^{b}, indépendamment, est H, alkyle, aryle, hétéroaryle; et chacun des R^{c} et R^{d}, indépendamment, est H, alkyle, aryle, hétéroaryle, cyclyle, hétérocyclyle, ou alkylcarbonyle,
où alkyle désigne un groupe alkyle contenant jusqu'à 6 atomes de carbone, alcényle désigne un groupe alcényle contenant jusqu'à 6 atomes de carbone, et alcynyle désigne un groupe alcynyle contenant jusqu'à 6 atomes de carbone, et
où les groupes alkyle, alcényle, alcynyle, aryle, hétéroaryle, cyclyle, et héthérocyclyle sont éventuellement substitués.

2. Composé selon la revendication 1, dans lequel U est N et V est CH.

3. Composé selon la revendication 1, dans lequel Z est N et W est O.

4. Composé selon la revendication 1, dans lequel X est O ou NR^{c}.

5. Composé selon la revendication 1, dans lequel Y est une liaison covalente, O, S, ou CH₂, et n est 0, 1, 2, 3, ou 4.

6. Composé selon la revendication 5, dans lequel R₃ est aryle, hétéroaryle, OR^{c}, SR^{c}, C(O)OR^{c}, NR^{c}R^{d}, ou C(O)NR^{c}R^{d}.

7. Composé selon la revendication 5, dans lequel R₃ est 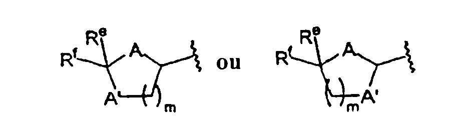 dans lequel
chacun des A et A¹, indépendamment, est O, S, ou NH;
chacun des R^{e} et R^{f}, indépendamment, est H, alkyle, aryle ou hétéroaryle; et
m est 1 ou 2,
tandis que alkyle désigne un groupe alkyle contenant jusqu'à 6 atomes de carbone, et tandis que les groupes alkyle, aryle, et hétéroaryle sont éventuellement substitués.

8. Composé selon la revendication 1, dans lequel l'un des R^{a} et R^{b} est 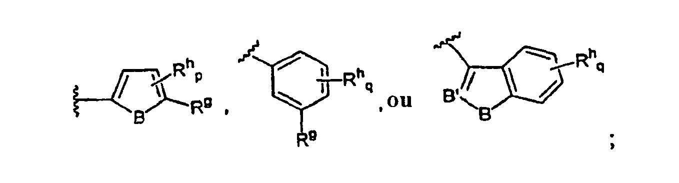 où
B est NRⁱ, O, ou S;
B' est N ou CRⁱ;
R^{g} est H, halogéno, CN, alkyle, cyclyle, alkyloxy, alkylcarbonyle, alkyloxycarbonyle, aryloxycarbonyle, hétéroaryloxycarbonyle, hydroxyalkyle, alkylamino, ou alkylaminocarbonyle;
R^{h} est H, halogéno, NO₂, CN, alkyle, aryle, hétéroaryle, OR^{c}, OC(O)R^{c}, SO₂R^{c}, S(O)R^{c}, S(O²)NR^{c}R^{d}, SR^{c}, NR^{c}R^{d}, NR^{c}COR^{d}, NR^{c}C(O)OR^{d}, NR^{c}C(O)NR^{c}R^{d}, NR^{c}SO₂R^{d}, COR^{c}, C(O)OR^{c}, ou C(O)NR^{c}R^{d};
Rⁱ est H, alkyle, ou alkylcarbonyle;
p est 0, 1, ou 2; et
q est 0, 1, 2, 3, ou 4,
tandis que alkyle désigne un groupe alkyle contenant jusqu'à 6 atomes de carbone, et
tandis que les groupes alkyle, aryle, hétéroaryle et cyclyle sont éventuellement substitués.

9. Composé selon la revendication 8, dans lequel l'un des R^{a} et R^{b} est 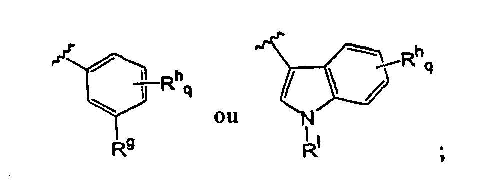 où
R^{g}, R^{h}, Rⁱ, et q sont tels que définis dans la revendication 8; et
l'autre des R^{a} et R^{b} est H ou alkyle.

10. Composé selon la revendication 9, dans lequel
R^{g} est H, méthyle, éthyle, propyle, cyclopropyle, méthoxy, éthoxy, méthoxycarbonyle, ou halogéno;
R^{h} est F, Cl, CN, méthyle, méthoxy, éthoxy, OC(O)CH₃, OC(O)C₂H₅, C(O)OH, C(O)OC₂H₅, C(O)NH₂, NHC(O)CH₃, ou S(O₂)NH₂;
Rⁱ est H, méthyle, éthyle, ou acétyle; et
q est 0, 1, ou 2.

11. Composé selon la revendication 10, dans lequel U est N, V est CH, Z est N, et W est O.

12. Composé selon la revendication 11, dans lequel X est NR^{c}; et R^{c} est H, méthyle, éthyle, ou acétyle.

13. Composé selon la revendication 12, dans lequel Y est une liaison covalente, O, S, ou CH₂; et n est 0,1, 2, 3, ou 4.

14. Composé selon la revendication 13, dans lequel R₃ est R^{c}, OR^{c}, SR^{c}, C(O)OR^{c}, ou C(O)NR^{c}R^{d}.

15. Composé selon la revendication 14, dans lequel R₃ est aryle, hétéroaryle, hydroxyle, alkyloxy, ou hétéroaryloxy.

16. Composé selon la revendication 1, dans lequel
chacun des R₂ et R₄ est H;
R₃ est H, alkyle, aryle, hétéroaryle, cyclyle, hétérocyclyle, alkyloxycarbonyle, alkylaminocarbonyle, ou alkylcarbonyle; et
X est NR^{c}.

17. Composé selon la revendication 16, dans lequel X est NH.

18. Composé selon la revendication 16, dans lequel chacun des R^{a} et R^{b} est H ou alkyle; et l'autre est aryle ou hétéroaryle éventuellement substitué par R^{g}_{q} et R^{h}; R^{g} étant un halogéno, CN, alkyle, alkyloxy, alkylcarbonyle, alkyloxycarbonyle, aryloxycarbonyle, hétéroaryloxycarbonyle, hydroxyalkyle, alkylamino, ou alkylaminocarbonyle; R^{h} étant halogéno, CN, hydroxyle, alkyle, aryle, hétéroaryle, alcoxyle, aryloxyle, ou hétéroaryloxyle; et q étant 0, 1, 2, 3, ou 4.

19. Composé selon la revendication 18, dans lequel chacun des R^{a} et R^{b} est
H ou alkyle; et l'autre est 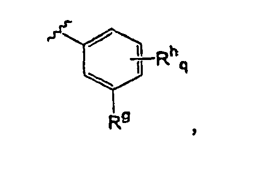 où
R^{g} est H, alkyle, alcoxyle, méthoxycarbonyle, ou halogéno;
R^{h} est halogéno, CN, hydroxyle, alkyle, aryle, hétéroaryle, alcoxyle, aryloxyle, ou hétéroaryloxyle; et
q est 0,1, 2, 3, ou 4,
tandis que alkyle désigne un groupe alkyle contenant jusqu'à 6 atomes de carbone, et
tandis que les groupes alkyle, aryle, et hétéroaryle sont éventuellement substitués.

20. Composé selon la revendication 16, dans lequel U est N, V est CH, Z est N, et W est O.

21. Composé selon la revendication 20, dans lequel R₃ est hétéroaryle ou hétérocyclyle.

22. Composé selon la revendication 21, dans lequel R₃ est pyridinyle, 1-oxy-pyridinyle, ou 1*H*-pyridine-2-one.

23. Composé selon la revendication 21, dans lequel n est 2, et Y est O.

24. Composé selon la revendication 23, dans lequel X est NH.

25. Composé selon la revendication 24, dans lequel l'un des R^{a} et R^{b} est H
ou alkyle; et l'autre est 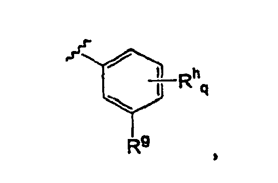 où
R^{g} est H, alkyle, alcoxyle, méthoxycarbonyle, ou halogéno;
R^{h} est halogéno, CN, hydroxyle, alkyle, aryle, hétéroaryle, alcoxyle, aryloxyle, ou hétéroaryloxyle; et
q est 0, 1, 2, 3, ou 4,
tandis que alkyle désigne un groupe alkyle contenant jusqu'à 6 atomes de carbone, et
tandis que les groupes alkyle, aryle, et hétéroaryle sont éventuellement substitués.

26. Composé selon la revendication 25, dans lequel l'un des R^{a} et R^{b} est H; et l'autre est 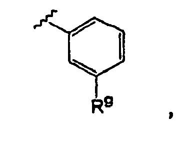 où R^{g} est tel que défini dans la revendication 25.

27. Composé selon la revendication 1, dans lequel le composé est:
N-{2-[3-(3,4-diméthoxy-phényl)-propyl]-6-morpholine-4-yl-pyrimidine-4-yl}-N'-(1H-indole-3-ylméthylène)-hydrazine,
N-(2-n-butoxy-6-morpholine-4-yl-pyrimidine-4-yl)-N'-(1H-indole-3-ylméthylène)-hydrazine,
N-(2-(4-hydroxybutoxy)-6-morpholine-4-yl-pyrimidine-4-yl)-N'-(1H-indole-3-ylméthylène)-hydrazine,
N-[2-(2-[1,3]dioxane-2-yl-éthyl)-6-morpholine-4-yl-pyrimidine-4-yl]-N'-(1H-indole-3-ylméthylène)-hydrazine,
N-(1H-indole-3-ylméthylène)-N'-[2-(3-méthoxy-propyl)-6-morpholine-4-yl-pyrimidine-4-yl]-hydrazine,
3-{4-[N'-(1H-indole-3-ylméthylène)-hydrazino]-6-morpholine-4-yl-pyrimidine-2-ylsulfanyl}-propane-1-ol,
3-{2-[N'-(1H-indole-3-ylméthylène)-hydrazino]-6-morpholine-4-yl-pyrimidine-2-ylsulfanyl}-propane-1-ol,
N-[2-(2,2-diméthyl-[1,3]dioxolane-4-ylméthoxy)-6-morpholine-4-yl-pyrimidine-4-yl]-N'-(1H-indole-3-ylméthylène)-hydrazine,
N-{2-[2-(3,4-diméthoxy-phényl)-éthoxy]-6-morpholine-4-yl-pyrimidine-4-yl}-N'-(1H-indole-3-ylméthylène-hydrazine,
N-(1H-indole-3-ylméthylène)-N'-[6-morpholine-4-yl-2-(2-pyridine-2-yl-éthoxy)-pyrimidine-4-yl]-hydrazine,
N-(1H-indole-3-ylméthylène)-N'-[6-morpholine-4-yl-2-(3-pyridine-2-yl-propyl)-pyrimidine-4-yl]-hydrazine,
N-(3-méthyl-benzylidène)-N'-[6-morpholine-4-yl-2-(2-pyridine-2-yl-éthoxy)-pyrimidine-4-yl]-hydrazine,
N-(3-éthyl-benzylidène)-N'-[6-morpholine-4-yl-2-(2-pyridine-2-yl-éthoxy)-pyrimidine-4-yl]-hydrazine,
N-(3-méthyl-benzylidène)-N'-[6-morpholine-4-yl-2-(3-pyridine-2-yl-propyl)-pyrimidine-4-yl]-hydrazine,
N-[6-morpholine-4-yl-2-(2-pyridine-2-yl-éthoxy)-pyrimidine-4-yl]-N'-(1-*m*-tolyl-éthylidène)-hydrazine,
N-[1-(1H-indole-3-yl)-éthylidéne]-N'-[6-morpholine-4-yl-2-(2-pyridine-2-yl-éthoxy)-pyrimidine-4-yl]-hydrazine,
3-méthyl-benzaldéhyde O-[6-morpholine-4-yl-2-(2-pyridine-2-yl)-éthoxy-pyrimidine-4-yl]-oxime,
1H-indole-3-carbaldéhyde O-[6-morpholine-4-yl-2-(2-pyridine-2-yl)-éthoxy-pyrimidine-4-yl]-oxime,
N-(1H-indole-3-ylméthyléne)-N'-{6-morpholine-4-yl-2-[2-(pyridine-3-yloxy)-éthoxy]-pyrimidine-4-yl}-hydrazine,
N-(3-méthyl-benzylidène)-N'-{6-morpholine-4-yl-2-[2-(pyridine-3-yloxy)-éthoxy]-pyrimidine-4-yl}-hydrazine,
butyl-{4-[N'-(1H-indole-3-ylméthylène)-hydrazino]-6-morpholine-4-yl-pyrimidine-2-yl}-hydrazine,
N-(3-méthyl-benzylidène)-N'-[6-morpholine-4-yl-2-(pyridine-3-yloxy)-pyrimidine-4-yl]-hydrazine,
N-(3-méthyl-benzylidène)-N'-(5-méthyl-6-morpholine-4-yl-2-phénylpyrimidine-4-yl)-hydrazine,
N-(3-méthyl-benzylidène)-N'-(2-phényl-6-thiomorpholine-4-yl-pyrimidine-4-yl)-hydrazine,
ester éthylique d'acide 3-{4-[N'-(3-méthyl-benzylidène)-hydrazino]-6-morpholine-4-yl-pyrimidine-2-yl}-propionique,
N-(3-méthyl-benzylidène)-N'-{6-morpholine-4-yl-2-[2-(1-oxypyrdine-2-yl)-éthoxy]-pyrimidine-4-yl}-hydrazine,
1-(2-{4-[N'-(3-méthyl-benzylidène)-hydrazino]-6-morpholine-4-yl-pyrimidine-2-yloxy}-éthyl)-1H-pyridine-2-one,
N-(3-iodo-benzylidène)-N'-[6-morpholine-4-yl-2-(2-pyridine-2-yl-éthoxy)-pyrimidine-4-yl]-hydrazine,
N-(3-fluoro-benzylidène)-N'-[6-morpholine-4-yl-2-(2-pyridine-2-yl-éthoxy)-pyrimidine-4-yl]-hydrazine,
N-(3-chloro-benzylidéne)-N'-[6-morpholine-4-yl-2-(2-pyridine-2-yl-éthoxy)-pyrimidine-4-yl]-hydrazine,
N-(3-bromo-benzylidène)-N'-[6-morpholine-4-yl-2-(2-pyridine-2-yl-éthoxy)-pyrimidine-4-yl]-hydrazine,
ester méthylique d'acide 3-{[6-morpholine-4-yl-2-(2-pyridine-2-yl-éthoxy)-pyrimidine-4-yl]-hydrazonométhyl}-benzoïque, 1-(2-{4-[N'-(3-iodo-benzylidène)-hydrazino]-6-morpholine-4-yl-pyrimidine-2-yloxy}-éthyl)-1H-pyridine-2-one,
amide N-méthylique d'acide 3-{[6-morpholine-4-yl-2-(2-pyridine-2-yl-éthoxy)-pyrimidine-4-yl]-hydrazonométhyl}-benzoïque,
(3-{[6-morpholine-4-yl-2-(2-pyridine-2-yl-éthoxy)-pyrimidine-4-yl]-hydrazonométhyl}-phényl)-méthanol,
N,N-diéthyl-4-{4-[N"-(3-méthyl-benzylidène)-hydrazino]-6-morpholine-4-yl-pyrimidine-2-yl}-butyramide,
4-{4-[N'-(3-méthyl-benzylidène)-hydrazino]-6-morpholine-4-yl-pyrimidine-2-yl}-1-(4-méthyl-pipérazine-1-yl)-butane-1-one,
4-{4-[N'-(3-méthyl-benzylidène)-hydrazino]-6-morpholine-4-yl-pyrimidine-2-yl}-N-pyridine-4-ylméthyl-butyramide, ou
4-{4-[N'-(3-méthyl-benzylidène)-hydrazino]-6-morpholine-4-yl-pyrimidine-2-yl}-N-pyridine-4-yl-butyramide.

28. Composition pharmaceutique comprenant un support pharmaceutiquement acceptable et une quantité efficace d'un composé selon l'une quelconque des revendications 1 à 27.

29. Utilisation d'un composé selon l'une quelconque des revendications 1 à 27 pour la préparation d'une composition pharmaceutique pour le traitement d'un trouble lié à la surproduction d'interleukine-12.

30. Utilisation selon la revendication 29, dans laquelle le trouble est la polyarthrite rhumatoïde, la septicémie, la maladie de Crohn, la sclérose en plaques, le psoriasis, ou le diabète insulino-dépendant.
